# EUROPEAN PATENT SPECIFICATION

(11) **EP 2 861 985 B1**
(45) Date of publication and mention of the grant of the patent: **15.11.2017**
(21) Application number: 13725576.6
(22) Date of filing: 28.05.2013
(51) Int. Cl.: G01N 33/50

(54) **PROTEIN EXPRESSION ANALYSES FOR IDENTIFYING GENOTOXIC COMPOUNDS**
PROTEINEXPRESSIONSANALYSEN ZUR IDENTIFIZIERUNG GENOTOXISCHER VERBINDUNGEN
ANALYSES DE L'EXPRESSION DE PROTÉINES POUR L'IDENTIFICATION DE COMPOSÉS GÉNOTOXIQUES

(30) Priority: 13.06.2012 US 201261659024 P
(43) Date of publication of application: 22.04.2015
(73) Proprietor: Merck Patent GmbH, 64293 Darmstadt (DE)
(72) Inventor: MUELLER, Stefan Otto, 64625 Bensheim (DE); DIETZ, Yasmin, 65462 Gustavsburg (DE)
(86) International application number: PCT/EP2013/001575
(87) International publication number: WO 2013/185884

(56) References cited:
- STÉPHANIE SOLIER ET AL: "The apoptotic ring", CELL CYCLE, vol. 8, no. 12, 15 June 2009 (2009-06-15), pages 1853-1859, XP055066877,
- M. MALMLOF ET AL: "Mdm2 as a Sensitive and Mechanistically Informative Marker for Genotoxicity Induced by Benzo[a]pyrene and Dibenzo[a,l]pyrene", TOXICOLOGICAL SCIENCES, vol. 102, no. 2, 18 October 2007 (2007-10-18), pages 232-240, XP055066879, ISSN: 1096-6080, DOI: 10.1093/toxsci/kfm305
- WEN-WEN CHOU ET AL: "Arecoline-induced phosphorylated p53 and p21 WAF1 protein expression is dependent on ATM/ATR and phosphatidylinositol-3-kinase in clone-9 cells", JOURNAL OF CELLULAR BIOCHEMISTRY, vol. 107, no. 3, 1 June 2009 (2009-06-01), pages 408-417, XP055066909, ISSN: 0730-2312, DOI: 10.1002/jcb.22137
- T. THOMPSON ET AL: "Phosphorylation of p53 on Key Serines Is Dispensable for Transcriptional Activation and Apoptosis", JOURNAL OF BIOLOGICAL CHEMISTRY, vol. 279, no. 51, 6 October 2004 (2004-10-06), pages 53015-53022, XP055067470, ISSN: 0021-9258, DOI: 10.1074/jbc.M410233200
- ZOE A STEWART ET AL: "Increased p53 phosphorylation after microtubule disruption is mediated in a microtubule inhibitor- and cell-speci c manner", ONCOGENE, vol. 20, 15 December 2000 (2000-12-15), pages 113-124, XP055067473,
- XIAOBING LIANG ET AL: "The Role of Wild-Type p53 in Cisplatin-Induced Chk2 Phosphorylation and the Inhibition of Platinum Resistance with a Chk2 Inhibitor", CHEMOTHERAPY RESEARCH AND PRACTICE, vol. 4, no. 3, 1 January 2011 (2011-01-01) , pages 216-8, XP055067516, ISSN: 2090-2107, DOI: 10.1158/1535-7163.MCT-08-0492
- Y. ZHU ET AL: "Intra-S-Phase Checkpoint Activation by Direct CDK2 Inhibition", MOLECULAR AND CELLULAR BIOLOGY, vol. 24, no. 14, 29 June 2004 (2004-06-29) , pages 6268-6277, XP055067572, ISSN: 0270-7306, DOI: 10.1128/MCB.24.14.6268-6277.2004
- S. S. PALII ET AL: "DNA Methylation Inhibitor 5-Aza-2'-Deoxycytidine Induces Reversible Genome-Wide DNA Damage That Is Distinctly Influenced by DNA Methyltransferases 1 and 3B", MOLECULAR AND CELLULAR BIOLOGY, vol. 28, no. 2, 8 November 2007 (2007-11-08), pages 752-771, XP055067587, ISSN: 0270-7306, DOI: 10.1128/MCB.01799-07
- K. BOEHME ET AL: "Genomic Profiling Uncovers a Molecular Pattern for Toxicological Characterization of Mutagens and Promutagens In Vitro", TOXICOLOGICAL SCIENCES, vol. 122, no. 1, 28 April 2011 (2011-04-28), pages 185-197, XP055066869, ISSN: 1096-6080, DOI: 10.1093/toxsci/kfr090
- Jennifer T Fox ET AL: "Cell-based high-throughput screens for the discovery of chemotherapeutic agents", Oncotarget, vol. 3, no. 5, 29 May 2012 (2012-05-29), pages 581-585, XP055375006, DOI: 10.18632/oncotarget.513

## Description

The invention relates to a method for screening compounds with (pro-)genotoxic activity by providing a system being capable of expressing a panel of defined proteins, incubating at least a portion of the system with compounds to be screened, and comparing the expression of the proteins in the system with the protein expression in a control system, thereby detecting the (pro-)genotoxic activity. Another object of the invention concerns a method for monitoring the likelihood of developing a physiological and/or pathological condition, which is caused, mediated and/or propagated by the genetic deregulation of proliferation, differentiation and/or damage repair, in response to a compound administered to a mammal in need of such treatment by determining an expression level of defined proteins in a biological sample withdrawn from the mammal. The invention also relates to a use of a kit for screening compounds with (pro-)genotoxic activity comprising antibodies that specifically bind to marker proteins.
A comprehensive evaluation of a compound's genotoxic potential is an important part during chemical risk assessment and pharmaceutical development (cf Fox et al. 2012). The current standard testing battery involves in-vitro and in-vivo tests designed to detect compounds that induce DNA damage by a variety of different mechanisms of action. A bacterial reverse mutation assay as well as a genotoxicity test with mammalian cells in in-vitro and/or in-vivo is required. Genotoxicity testing systems gain in importance because of the fact that compounds tested positive in these tests are potential human carcinogens. However, the standard testing battery has a low throughput and need a comparatively large amount of compound. For this reason, these assays are less suitable in the early drug discovery phase (Westerink et al., 2011, Mutat. Res. 724, 7-21).
Frequently, chemicals that turn out not to be in-vivo genotoxins, or not to induce tumors via a mutagenic or DNA-reactive mechanism, give false positive results in mammalian cell tests, which complicate human hazard extrapolation.
Additionally, in terms of the chemical characterization within the EU REACH legislation and modern risk evaluation, mechanistic analyses were getting more important.
The difficulties are now forcing the development of new in-vitro tools. Novel genotoxicity test systems with enhanced specificity (i.e. less false positive results) without losing the sensitivity (i.e. detecting DNA-reactive carcinogens and in-vivo genotoxins) are needed (Kirkland et al., 2005, Mutat. Res. 584, 1-256; Kirkland et al., 2006, Mutat. Res. 608, 29-42; Kirkland et al., 2008, Mutat. Res. 653, 99-108).
Therefore, the technical problem forming the basis of the present invention is to provide a method for screening compounds, which effectively allows the identification and characterization of their genotoxic and/or pro-genotoxic properties. It is another problem of the invention to provide a use of a kit for the detection of genotoxic and/or pro-genotoxic activity.
The present invention solves the problem by providing a method for screening compounds with genotoxic and/or pro-genotoxic activity comprising the step of determining expression levels of active forms of the proteins p-p53 (Ser15), p21, p-H2A.X (Ser139), p-ATM (Ser1981), p-Chk1 (Ser345), in a system incubated with one or more compounds in comparison with expression levels of said proteins in the system substantially not incubated with the compounds or the same concentration of the compounds.

It has been surprisingly demonstrated by the inventors that the combination of at least five of the said proteins is correlated with genotoxicity. Consequently, the aforementioned plurality of marker proteins represents novel genotoxicity markers, which are well suited for differentiating the stage of genotoxicity. The underlying proteins are selected as result of differential expression analysis. All protein expressions are characterized by a distinct difference to untreated cells, wherein the treated cells show the difference by up-regulation. The determined proteins are already described in the art by sequence and other features, but lacking a linkage to genotoxicity in the defined combination of the invention. Either of the pairwise marker protein combinations, optionally supplemented with further marker proteins, can be used for the utmost test reliability. The proteins may be named in another way, but are uniquely defined by the accession number, which is generally accepted and registered in numerous data bases, such as the UniProt, SwissProt and the like (cf. Table 1; Zhou and Bartek, 2004, Nat Rev Cancer 4, 216-25).
Although the identified nine proteins are involved in DNA-damage-response signal-transduction network, they are not inevitably associated by function or location in their entity as presently known, but it is not excluded that such relations appear between a single member or more members of the group.

The DNA-damage-response signal-transduction network is composed of at least two different pathways: Ataxia telangiectasia mutated kinase (ATM) and ataxia telangiectasia-mutated and Rad3-related (ATR) pathway. In response to damaged DNA, the ATM pathway is initiated by autophosphorylation of ATM on Ser1981. ATM regulates cell cycle checkpoints and DNA repair via phosphorylation of a couple of proteins. The ATR pathway responds to DNA-double strand breaks (DSB) - although slower than ATM - and to chemicals that interfere with the DNA replication fork. There are two phospho-sites (p-ATR (Ser428) and p-ATR (Thr1989)) which may lead to the activation of ATR. The sensors that initially recognize aberrant DNA structure and activate the DNA-damage-response network are unclear.

A very early event of DNA-damage response is the phosphorylation of histone H2A.X via ATM at Ser139. Phosphorylated H2A.X accumulates at sites of DNA damage and recruits a variety of DNA-damage response and DNA repair signals.

DNA double-strand breaks induce Chk2 phosphorylation at Thr68 and other sites in this region by ATM which leads to p53-dependent or p53-independent cell cycle arrest and/or apoptosis.

The phosphorylation of p53 at position Ser15 in particular via ATM prevents the ability of mdm2 to bind and detains its degradation. Phosphorylated and therefore activated p53 acts as transcription factor for a couple of genes including the gene for the protein p21 and growth arrest and DNA damage-inducible protein a (Gadd45a). P21 inhibits different cyclin-dependant kinases which lead to a cell cycle arrest in G1-phase. Furthermore, p21 binds the PCNA and interrupts the DNA-replication. Induced nuclear Gadd45a interacts with the cyclin-dependant kinase 2 (cdc2) and leads to its dissociation from the cdc2/cyclin B1 complex. Free cyclin B1 is exported into the cytoplasm and degraded via ubiquitination. Gadd45a therefore inhibits the cdc2 kinase activity and the cell remains at the transition into mitosis.

Besides the Gadd45a-mediated inhibition of the cdc2 kinase, mediation via Chk1 also occurs. Chk1 can be activated by the kinases ATM and ATR, but the phosphorylation of Chk1 at Ser345 and Ser317 are performed by ATR. Chk1 is able to phosphorylate the phosphatase Cdc25C at Ser216 which disables the loss inhibitory phosphorylation of cdc2 at Thr14 and Tyr15 and the cell remains at the G2/M phase transition.

The analysis of the proteins and associated molecules, as discussed above, demonstrates the complex regulation of cell death and survival, e.g. in response to the exposure of HepG2 cells with (pro-)genotoxicants. Mainly affected processes comprise cell cycle regulation, cell proliferation and apoptosis. Proteins found to be differentially regulated can be predominantly assigned to pro-apoptotic and anti-proliferative functions. The data indicate an induction of cell cycle arrest and programmed cell death in response to compound-induced DNA damage.

The linkage of genotoxicity to distinct proteins is utilized for the in-vitro detection of mutagens and pro-mutagens, which are able to interfere with signaling in proliferation, differentiation or damage repair. Building a compound-specific protein expression profile, which is based on the plurality of proteins of the invention, is of unexpected benefit in establishing a genotoxic mechanism of action and, therefore, supports the evaluation of potential hazards or benefits of novel compounds either alternatively or supplementary to the classical screening methods. The inventive principle underlying the present method comprises detecting the defined proteins. The gene product is chosen in respect of both its absolute and relative amount as well as the specificity for a certain cell type.

A "protein" refers to a biochemical compound comprising one or more polypeptides. A polypeptide is a single linear polymer chain of amino acids bonded together by peptide bonds between the carboxyl and amino groups of adjacent amino acid residues. It is typically folded into a globular or fibrous form to facilitate a biological function. An "active form" of a protein refers to the fact that the residues in a protein are often chemically modified by posttranslational modification, which alters the physical and chemical properties, folding, stability, activity, and ultimately, the function of the protein. For example, the activity of the transcription factor p53 is crucially regulated by means of protein phosphorylation which can be induced by multiple signals, including general cellular stress, DNA damage or interferons.

A "plurality of proteins" as used herein refers to a group of identified or isolated proteins whose levels of expression vary in different tissues, cells or under different conditions or biological states. The different conditions may be caused by exposure to certain agent(s) - whether exogenous or endogenous - which include hormones, receptor ligands, chemical compounds and the like. The expression of a plurality of proteins demonstrates certain patterns. That is, each protein in the plurality is expressed differently in different conditions, or with or without exposure to certain endogenous or exogenous agents. The extent or level of differential expression of each protein vary in the plurality and may be determined qualitatively and/or quantitatively according to this invention. A protein expression profile, as used herein, refers to a plurality of proteins that are differentially expressed at different levels, which constitutes a "pattern" or a "profile." As used herein, the term "expression profile", "profile", "expression pattern", "pattern", "protein expression profile" and "protein expression pattern" are used interchangeably.
As used herein, a "compound with genotoxic activity", also referred to as "mutagen", is a physical or chemical agent that changes the genetic material, usually DNA, of an organism and thus increases the frequency of mutations above the natural background level. The skilled artisan would know that, for instance, one of the biological effects of mutagens is to promote the development of cancer. Other biological effects of mutagens are well documented and discussed. The changes in nucleic acid sequences by mutations comprise substitution of nucleotide base-pairs and insertions and deletions of one or more nucleotides in DNA sequences.
First, a system is provided and incubated in total or in part(s). The system is also referred to as test system hereunder. Although a cellular system is preferred an in-vitro translation system can be alternatively used which is based on the protein synthesis without living cells. The cellular system is defined to be any subject provided that the subject comprises cells. Hence, the cellular system can be selected from the group of single cells, cell cultures, tissues, organs, plants and animals. The scope of the cellular system also comprises parts of such biological entities, i.e. samples of tissues, organs, plants and animals. Barring plants, it shall be understood that each cellular system in the aforementioned order could represent a sample of the respective following system. Cell samples, non-human animals, mammals or non-human mammals are preferred embodiments of the cellular system according to the invention. A cell sample refers to any type of primary cells or genetically engineered cells, either in the isolated status, in culture or as cell line. Particularly, the cell sample is taken in-vivo or in-situ from a mammal to be tested. The withdrawal of the cell sample follows good medical practice. Biological samples may be taken from any kind of biological species, but the sample is especially taken from a human, rat or a mouse. The cellular system may also comprise a biological fluid, wherein the sample of body fluid preferably consists of blood, serum, plasma, saliva or urine. It is also preferred to gather a tissue sample by biopsy, especially taken close to the location of ailment. The biological samples can be originated from any tissue, including the liver, kidney, intestine, bone marrow, etc. In preferred embodiments, the biological samples are from the kidney, liver, and the intestine. The sample may be purified to remove disturbing substances, such as inhibitors for the formation of hydrogen bonds.
The system is capable of expressing, or expressing, several proteins of p53, p21, H2A.X, ATM, Chk1, ATR, cdc2, Gadd45a or Chk2. The system is additionally capable of expressing, or expressing, MDM2. The system is also capable of activating, or activating, several proteins of p-p53 (Ser15), p21, p-H2A.X (Ser139), p-ATM (Ser1981), p-Chk1 (Ser345), p-ATR (Ser428), p-cdc2 (Thr14/Tyr15), Gadd45a and p-Chk2 (Thr68). The system is preferably capable of expressing, or expressing, active forms of at least two proteins selected from the group of p-p53 (Ser15), p21, p-H2A.X (Ser139), p-ATM (Ser1981), p-Chk1 (Ser345), p-ATR (Ser428), p-cdc2 (Thr14/Tyr15), Gadd45a and p-Chk2 (Thr68). More preferably, the system is capable of expressing, or expressing, active forms of at least two proteins selected from the group of p-p53 (Ser15), p21, p-H2A.X (Ser139) and p-Chk1 (Ser345). Most preferably, the system is capable of expressing, or expressing, active forms of at least two proteins selected from the group of p-p53 (Ser15), p21, p-H2A.X (Ser139), p-ATM (Ser1981) and p-Chk1 (Ser345).
It shall be understood that variants, mutants, parts or homologous protein sequences having the same function, are included in the description. Preferably, the homology amounts to at least 85 %. Possible alterations comprise deletion, insertion, substitution, modification and addition of at least one amino acid, or the fusion with another peptide or protein. The engineered cells are capable of expressing these proteins after transfection with appropriate vectors harboring the underlying nucleic acid sequence or parts thereof. Preferably, the recombinant cells are of eukaryotic origin.

In a more preferred embodiment of the present invention, HepG2 cells are provided for the screening method. The dose of S9 used has an influence on the experimental outcome in spite of being negative in cytotoxicity evaluations. It should be noted that the dose is comparable to the dose used within regulatory standard genotoxicity testing. The significantly S9-regulated genes differ from those induced by the (pro-)genotoxic agents and the S9-effect can be adjusted by comparison to the proper controls. In any case, a cellular system having sufficient intrinsic metabolic activity would certainly be appropriate, but currently no such metabolically competent cellular system exists for genotoxicity evaluation. Primary hepatocytes are the current gold standard for drug metabolism and CYP induction/ inhibition studies in-vitro. Even though adult differentiated hepatocytes lack proliferation competency making them an unsuitable model for genotoxicity investigations, and they also exhibit significant changes in morphology as well as protein and gene expression during cultivation, limited availability of human hepatocytes and often marked donor/batch variability complicating standardization of such systems, a major advantage of HepG2 cells are their human molecular characteristics. For instance, specific targets such as topoisomerases and eukaryotic repair enzymes are expressed and prevent the overestimation of genotoxicity and therefore, contribute to a reduction of false positives.

The cell sample is stored, such as frozen, cultivated for a certain period or immediately subjected to the next step. Before incubating it with compounds to be screened, the cell sample could be divided into multiple portions. If doing so, at least two portions are provided; one is used for screening while the other one serves as control. Preferably, the number of portions for screening exceeds the number of control portions. Usually, numerous portions are subjected to a high-throughput screening.

The compounds are composed of biological and/or chemical structures capable to interact with a target molecule. Herein, any component of genomics or proteomics signaling shall be considered as "target molecule", which is not limited to genes, or a regulator protein or a gene product thereof, or a component of a signal transduction pathway comprising a gene or gene products thereof. Consequently, the specific interaction of compounds may involve either the mere targeting or the induction of alterations in cell function, or it may even include both effects simultaneously.

The compounds to be screened in the inventive method are not restricted anyway. In particular, the compounds are selected from the group of nucleic acids, peptides, carbohydrates, polymers, small molecules having a molecular weight between 50 and 1.000 Da and proteins. These compounds are often available in libraries. It is preferred to incubate a single compound within a distinct portion of the cell sample. However, it is also possible to investigate the cooperative effect of compounds by incubating at least two compounds within one portion. While a fraction of the cellular system is incubated with one or more (pro-)genotoxic compounds to be analyzed, a further portion of cells is incubated in the absence of the compounds and this additional non-treated fraction of the system is used as negative control. It is also possible that the system acts simultaneously as test and control system by determining the status before exposing genotoxic stress and comparing it with the status upon genotoxic stress. Further control systems are outlined below.

The term "incubation" denotes the contacting of the compounds with the cells for a distinct period, which depends on the kind of compounds and/or target. The incubation process also depends on various other parameters, e.g. the cell type and the sensitivity of detection, which optimization follows routine procedures known to those skilled in the art. The incubation procedure can be realized without a chemical conversion of mutagens or may involve a metabolic conversion of pro-mutagens. Adding chemical solutions and/or applying physical procedures, e.g. impact of heat, can improve the accessibility of the target structures in the sample. Specific incubation products are formed as result of the incubation.
The identification of effective compounds in the meaning of the invention is indirectly performed by determining the expression pattern of at least five defined proteins, which the system is capable of expressing. The determination is performed at a specified moment and correlated to the signal strength at the beginning of the experiment and the control. For example, the control system is not incubated with the compounds (negative control), or the control system is incubated with a standard compound having no genotoxic activity (negative control). The control system can also be incubated with a standard compound having (pro-)genotoxic activity (positive control). The activity is revealed by a change in expression. Preferably, the proteins expressed in cells with mutagen exposure are compared to the proteins expressed in cells that were not exposed to mutagens. Pairwise comparisons are made between each of the treatments. A pairwise comparison involves that the expression data for a given protein under a given treatment condition are compared to the expression data for this protein under a second treatment condition. The comparison is performed using suitable statistical technique with the assistance of known and commercially available programs.
It is another preferred aspect of the invention that the inherent (pro-)genotoxic activity is detected if the expression of proteins is up-regulated in the system in comparison with a negative, positive or negative control system, or if the expression of proteins is substantially identical in the system and a positive control system. In detail, (i) a higher protein expression in the test system in comparison with a negative control system, which is not incubated with any compound, indicates said activity, or (ii) a substantially identical or higher protein expression in the test system in comparison with a positive control system, which is incubated with a standard compound having genotoxic and/or pro-genotoxic activity, indicates said activity, or (iii) a higher protein expression in a relative control system, which is incubated with compounds having a concentration other than that in the test system with the proviso that a lower concentration is assigned to the relative control system, indicates said activity. It shall be understood that it is not required to determine the expression by five or all comparisons set forth above but the indication of (pro-)genotoxic activity resulting from any comparison of expression levels under (i), (ii) or (iii) inevitably implies said activity in any other comparative determination (whether performed or not).
It is more preferred that an increase in the expression levels of at least one of said proteins in the system incubated with the compounds in comparison with the system not incubated with the compounds indicate said activity. It is most preferred embodiment of the invention that the expression levels of at least five, six, seven, eight or nine of said proteins are increased. Highly preferably, the existing activity is detected by differential protein expression analysis with the negative control system.
In another preferred aspect of the invention, the expression levels are increased by a factor of at least 1.5, more preferably at least 1.6, most preferably at least 1.8, highly preferably at least 1.9, and particularly highly preferably at least 2.1.

It is another most particular aspect that a decreased concentration of the compounds is administered to provide the expression levels having a factor from 1 to 1.1 compared to those of the control system. The decreased compound concentration refers to a decrease of at least 10%, preferably at least 20%, more preferably at least 30%, most preferably at least 40%, highly preferably at least 50%, particularly highly preferably at least 60%.

In another more preferred aspect, substantially identical expression levels of at least one of said proteins in the system incubated with the compounds in comparison with the system incubated with a standard compound having genotoxic and/or pro-genotoxic activity indicate said activity.
Suitable tests for monitoring protein expression, determination and variant analysis of amino acid sequences are known to those skilled in the art or can be easily designed as a matter of routine. The assay according to the invention may be any assay suitable to detect and/or quantify protein expression. It is a preferred embodiment of the invention that the expression levels are determined by immunofluorescence staining.
The selected markers can be particularly used to establish screening tools with a higher throughput, preferably High Content Imaging (HCl) or Luminex xMAP technology. Luminex color-codes tiny beads, called microspheres, into 500 distinct sets. Each bead set can be coated with a reagent specific to a particular bioassay, allowing the capture and detection of specific analytes from a sample. The Luminex technology particularly combines a sandwich ELISA immobilized on microparticle beads and flow cytometry. It allows simultaneous quantitative measurement of several proteins in one single sample. Inside the Luminex analyzer, a light source excites the internal dyes that identify each microsphere particle, and also any reporter dye captured during the assay. Dual lasers are employed to detect identity of the beads (based on the spectral properties of the beads specific for each analyte) and the amount of associated Phycoerythrin (PE) fluorescence (Hoffmann et al, 2010, Toxicology 277, 49-58). Automated imaging platforms combining fluorescence microscopy with image analyses algorithms and informatics tools enable the analyses of fluorescent images from millions of cells with a high-resolution examination of the localization of cellular components, cellular macromolecular structures and the temporal dynamics of cellular functions. The application of the HCl technology for toxicological evaluation shows that in-vitro predictions can fortify the performance of traditional preclinical tests by identifying human hepatotoxicants (Xu et al., 2008, Toxicol Sci 105, 97-105). Furthermore, the HCl technology can be used for genotoxicity evaluation by detecting micronuclei (MN). The in-vitro MN test detects compound-induced clastogenic (chromosome damage) and aneugenic (chromosome loss) effects, by counting the chromosomal fragments called micronuclei in the cytoplasm of the cells. A HCl-based assay is able to detect genotoxic potential with a high performance score and also allow the differentiation between aneugenic and clastogenic compounds (Westerink et al., 2011, Mutat. Res. 724, 7-21).

Moreover, the technology allows the combination of several selected endpoints, preserving biological complexity and molecular interactions to a certain extent. HCl offers the possibility to combine classical genotoxic endpoints (e.g. MN induction) and the analysis of cellular markers with the simultaneous acquisition of cell viability/cytotoxicity. Cell viability is an important parameter to consider for genotoxicity testing because false positives in standard assays can be generated among others via cytotoxicity. The same holds true for measuring molecular marker, such as p53. Although p53 reacts extremely sensitive to DNA damage, nutrient deprivation and hypoxia could also induce activation. Consideration of cytotoxicity for dose selection, together with multiple endpoint measurements may prevent or reduce false positives.

Consequently, this invention relates to a method for predicting the cellular effect of a compound having genotoxic activity by preparing a protein sample from a cell to be evaluated, contacting the protein sample with an antibody, detecting a protein binding to the antibody, and comparing a detected result with a result detected using a protein sample prepared from a control cell.

The detection may be performed by applying the intact cell to a detection method of choice. It is preferred, however, to provide cellular extracts first. Cell lysis can be performed in suitable, well-known lysis buffers, which may cause an osmotic shock and perforate the cell membrane. The stability of the cell structure can also be destroyed by mechanical forces, such as ball mill, French press, ultrasonic, etc., by enzymatic degradation of cell wall and cell membrane, respectively, and/or by the action of tensides. The biomarkers may be further purified to remove disturbing substances or the biomarkers can be concentrated in the sample. Downstream-processing and/or concentrating are preferably performed by the method of precipitation, dialysis, gel filtration, gel elution, or chromatography, such as HPLC or ion exchange chromatography. It is recommended to combine several methods for better yields.

Suitable tests for detecting genotoxicity are known to those skilled in the art or can be easily designed as a matter of routine. Many different types of assays are known, examples of which are set forth below. Although the assay according to the invention may be any assay suitable to detect and/or quantify gene expression, genotoxicity is preferably determined by means of substances specifically interacting with the proteins p-p53 (Ser15), p21, p-H2A.X (Ser139), p-ATM (Ser1981), p-Chk1 (Ser345), p-ATR (Ser428), p-cdc2 (Thr14/Tyr15), Gadd45a and p-Chk2 (Thr68).

The term "specific substances" as used herein comprises biological and/or chemical structures capable to interact with the proteins of the invention in such a manner that makes a recognition, binding and interaction possible. In particular, the substances are selected from the group of nucleic acids, peptides, carbohydrates, polymers, small molecules having a molecular weight between 50 and 1.000 Da and proteins, preferably nucleic acids and proteins. The specific substances express a sufficient sensitivity and specificity in order to ensure a reliable binding and detection. Other biomolecules present in the sample do not significantly bind to the substance specific for the protein marker of the invention. Preferably, the level of binding to a biomolecule other than the marker protein results in a binding affinity of only 10 % of the affinity of the marker protein, more preferably only 5 % or less. A specific substance has at least an affinity of 10⁻⁷ M for the corresponding marker protein. The specific substance preferably has an affinity of 10⁻⁸ M or even more preferred of 10⁻⁹ M for the marker protein. A most preferred specific substance will fulfill both the above minimum criteria for affinity as well as for specificity.

The substances are preferably specific to parts of the protein. Such parts represent a restriction to those regions which are sufficient for the expression of a specific function, i.e. the provision of a structural determinant for recognition. All truncations are inevitably limited by the requirement of preserving the function, e.g. unique recognition. However, the protein fragments can be very small. Preferably, the substances are mono-specific in order to guarantee an exclusive and directed interaction with the chosen single target.

The recognition of the proteins according to the invention can be realized by a specific interaction with substances on the primary, secondary and/or tertiary structure level of an amino acid sequence. In the context of the present invention, the term "recognition" - without being limited thereto - relates to any type of interaction between the specific substances and the target, particularly covalent or non-covalent binding or association, such as a covalent bond, hydrophobic/ hydrophilic interactions, van der Waals forces, ion pairs, hydrogen bonds, ligand-receptor interactions, interactions between epitope and antibody binding site, nucleotide base pairing, and the like. Such association may also encompass the presence of other molecules such as peptides, proteins or other nucleotide sequences.

The specific substances are preferably selected from the group consisting of antibodies, cytokines, lipocalins, receptors, lectins, avidins, lipoproteins, glycoproteins, oligopeptides, peptide ligands and peptide hormones. More preferably, antibodies are used as specific substance. "Antibody" denotes a polypeptide essentially encoded by an immunoglobulin gene or fragments thereof. According to the invention, antibodies are present as intact immunoglobulins or a number of well-characterized fragments. Fragments are preferably selected from the group consisting of F_{ab} fragments, F_{c} fragments, single chain antibodies (scFv), variable regions, constant regions, H chain (V_{H}), and L chain (V_{L}), more preferably F_{ab} fragments and scFv. Fragments, such as F_{ab} fragments and F_{c} fragments, can be produced by cleavage using various peptidases. Furthermore, fragments can be engineered and recombinantly expressed, preferably scFv.

DNA aptamers and RNA aptamers have been found to express a high affinity for a wide variety of target molecules. Target structures may comprise proteins, peptides and small molecules, such as organic dyes, nucleotides, amino acids, vitamins, alkaloids, etc. More preferred are RNA aptamers since the 2'-hydroxyl group available in RNA promotes a couple of intra- and intermolecular contacts, the latter being between molecules of the same sequence, different sequences, or between RNA and any other molecule which is not composed of RNA. These nucleic acid ligands can be identified by an efficient in-vitro selection procedure - the so-called SELEX process (systematic evolution of ligands by exponential enrichment). Since RNA is very susceptible to nucleolytic degradation in biological solutions, RNA aptamers should be chemically modified using phosphorothioates, locked nucleic acids, or Spiegelmers, for instance. L-RNA versions of aptamers called Spiegelmers are especially long-lived as they are essentially impervious to natural degradation processes. Because of their high affinity for a broad spectrum of structural targets, aptamers act very similar to antibodies. Aptamers can be synthesized using standard phosphoramidite chemistry. In addition, RNA aptamers having more than approximately 30 nucleotides can be favorably synthesized in large amounts by in-vitro transcription. Selection, synthesis, and purification of aptamers are well-known to those skilled in the art.

The specific substances can be labeled, in doing so the labeling depends on their inherent features and the detection method to be applied, i.e. the required sensitivity, ease of conjugation, stability requirements, and available instrumentation and disposal provisions. For the detection of specific incubation products, the applied methods depend on the specific incubation products to be monitored and are well-known to the skilled artisan. Preferred examples of suitable detection methods according to the present invention are luminescence, particularly fluorescence, furthermore VIS coloring and/or radioactive emission.

Luminescence concerns the emission of light as a result of chemiluminescence, bioluminescence or photoluminescence. Chemiluminescence involves the emission of visible light as a result of a chemical reaction, whereas bioluminescence requires the activity of luciferase. The presently preferred photoluminescence, which is also known as fluorescence stimulation, is caused by the absorption of photons, preferably provided by radiation, which is released again as photon with a shift in wavelength of 30 to 50 nm and within a period of approximately 10⁻⁸ seconds. The instruments for fluorescence detection include, but are not limited to typical benchtop fluorometers, fluorescence multi-well plate readers, fiber optic fluorometers, fluorescence microscopes and microchips/microfluidics systems coupled with fluorescence detection.

VIS coloring denotes the visualization of any achromatic substance in order to be visible to the naked eye. Preferably, the intensity of coloring is measured by a photometer.

Radioactive radiation of isotopes is measured by scintillation. The process of liquid scintillation involves the detection of beta decay within a sample via capture of beta emissions in a system of organic solvents and solutes referred to as the scintillation cocktail. The beta decay electron emitted by radioactive isotopes such as ³H, ¹⁴C, ³²P, ³³P and ³⁵S in the sample excites the solvent molecule, which in turn transfers the energy to the solute. The energy emission of the solute (the light photon) is converted into an electrical signal by a photo-multiplier tube within a scintillation counter. The cocktail must also act as a solubilizing agent keeping a uniform suspension of the sample. Gamma ray photons often arise as a result of other decay processes (series decay) to rid the newly formed nucleus of excess energy. They have no mass and produce little if any direct ionization by collision along their path. Gamma photons are absorbed for detection and quantization by one or more of three mechanisms: The Compton effect, the photoelectric effect and pair production. A favorable gamma decay isotope of the present invention is ¹²⁵I.

A labeling method is not particularly limited as long as a label is easily detected. A "labeled specific substance" is one that is bound, either covalently through a linker or a chemical bond, or non-covalently through ionic, van der Waals, electrostatic, hydrophobic interactions or hydrogen bonds, to a label such that the presence of the marker proteins may be detected by detecting the presence of the label bound to them.

The covalent linkage of an antibody to an enzyme may be performed by different methods, such as the coupling with glutaraldehyde. Both, the enzyme and the antibody are interlinked with glutaraldehyde via free amino groups, and the by-products of networked enzymes and antibodies are removed. In another method, the enzyme is coupled to the antibody via sugar residues if it is a glycoprotein, such as the peroxidase. The enzyme is oxidized by sodium periodate and directly interlinked with amino groups of the antibody. Other enzyme containing carbohydrates can also be coupled to the antibody in this manner, however sometimes a loss in activity is observed due to the oxidation, e.g. a diminished activity of alkaline phosphatase. Enzyme coupling may also be performed by interlinking the amino groups of the antibody with free thiol groups of an enzyme, such as β-galactosidase, using a heterobifunctional linker, such as succinimidyl 6-(N-maleimido) hexanoate.

Specific immunological binding of an antibody to a protein can be detected directly or indirectly. Direct labels include fluorescent or luminescent tags, metals, dyes, radionuclides, and the like, attached to the antibody. An antibody labeled with iodine-125 (¹²⁵I) can be used. A chemiluminescence assay using a chemiluminescent antibody specific for the protein marker is suitable for sensitive, non-radioactive detection of protein levels. An antibody labeled with fluorochrome is also suitable. Examples of fluorochromes include, without limitation, DAPI, fluorescein, fluorescein isothiocyanate (FITC), Oregon Green, Hoechst 33258, R-phycocyanin, green fluorescent protein (GFP), B-phycoerythrin, R-phycoerythrin, rhodamine, Texas red, tetrarhodimine isothiocynate (TRITC), Cy3, Cy5 and lissamine. Indirect labels include various enzymes well known in the art, such as horseradish peroxidase (HRP), alkaline phosphatase (AP), β-galactosidase, urease and the like. A horseradish-peroxidase detection system can be used, for example, with the chromogenic substrate tetramethylbenzidine (TMB), which yields a soluble product in the presence of hydrogen peroxide that is detectable at 450 nm. An alkaline phosphatase detection system can be used with the chromogenic substrate p-nitrophenyl phosphate, for example, which yields a soluble product readily detectable at 405 nm. Similarly, a ß-galactosidase detection system can be used with the chromogenic substrate o-nitrophenyl-ß-D-galactopyranosxde (ONPG), which yields a soluble product detectable at 410 nm. A urease detection system can be used with a substrate, such as urea-bromocresol purple. Auto-quenched fluorescent compounds are activated by tumor-associated proteases, enzymes, e.g. luciferase, nanoparticles, biotin, digoxigenin and the like.

In another preferred embodiment of the present invention, the aptamers are labeled with digoxigenin, biotin, chemiluminescence substances, fluorescence dyes, magnetic beads, metallic beads, colloidal particles, electron-dense reagents, enzymes, all of them are well-known in the art, or radioactive isotopes. Preferred isotopes for labeling nucleic acids in the scope of the invention are ³H, ¹⁴C, ³²P, ³³P, ³⁵S, or ¹²⁵I, more preferred ³²P, ³³P, or ¹²⁵I.

A variety of immunoassay techniques, including competitive and non-competitive immunoassays, can be used. The term "immunoassay" encompasses techniques including, without limitation, enzyme immunoassays (EIA), such as enzyme multiplied immunoassay technique (EMIT), enzyme-linked immunosorbent assay (ELISA), IgM antibody capture ELISA (MAC ELISA) and microparticle enzyme immunoassay (MEIA), furthermore capillary electrophoresis immunoassays (CEIA), radio-immunoassays (RIA); immunoradiometric assays (IRMA), fluorescence polarization immunoassays (FPIA) and chemiluminescence assays (CL). If desired, such immunoassays can be automated. Immunoassays can also be used in conjunction with laser induced fluorescence such as the Luminex technology. Liposome immunoassays, such as flow-injection liposome immunoassays and liposome immunosensors, are also suitable for use in the present invention. In addition, nephelometry assays, in which the formation of protein/antibody complexes results in increased light scatter that is converted to a peak rate signal as a function of the marker concentration, are suitable for use in the methods of the present invention.

In an embodiment of the present invention, antibodies are used as specific substances and the incubation products are detected by the labeling of the antibodies, preferably by ELISA, RIA, fluoro immunoassay (FIA), soluble particle immune assay (SPIA) or western blotting.

In a preferred embodiment of the invention, an ELISA is used for the detection of the incubation products. Component of ELISAs are enzymes which are bound to one partner of the immunological reaction. The tracer antigen (analyte derivative) of a marker protein is preferably labeled in the competitive ELISA using a single capture antibody (herein after referred to as primary), whereas the antibody is preferably labeled in the non-competitive ELISA preferably comprising the precipitation of the antigen-antibody complex by a second antibody (herein after referred to as secondary) which is directed to another epitope of said marker protein than the primary antibody. Complexes consisting of antigen and two antibodies are also called sandwich complexes. The detection comprises the subsequent enzymatic conversion of a substrate to a product, preferably a colored product, which is recognized by visual coloring, bioluminescence, fluorescence or the measurement of electrical signals (enzyme electrode). Favorable enzymes for labeling in the present invention are known to the skilled artisan, such as peroxidase (e.g. HRP), chloramphenicol acetyl transferase (CAT), green fluorescent protein (GFP), glutathione S-transferase (GST), luciferase, β-galactosidase and AP.

Additionally preferred are radioactive immunoassays utilizing radioactive isotopes which are either incorporated into an immune reagent during synthesis, or subsequently coupled to an immune reagent of the assay, preferably to an antibody. Preferred radioactive isotopes in the inventive method are ³H, ¹⁴C, ³²P, ³³P, ³⁵S, and ¹²⁵I, and more preferred ¹⁴C, ³⁵S and ¹²⁵I. A favorite method follows the competitive principle of binding. A constant amount of radioactive protein marker and a variable amount of said marker of the sample to be analyzed compete for a defined amount of antibody which is present in excess. The displacement of tracer is directly proportional to the marker concentration which can be evaluated by a calibration curve.

Antigens or antibodies, respectively, which are favorably labeled with fluorophores, are used in FIAs.

SPIA utilizes the color change of silver particle as result of agglutination. Neither a secondary antibody nor an indicator reaction are required making it particularly useful in the scope of the present invention. Similarly favorably is the latex agglutination test using antibodies which are bound to colored latex particles.

Another favorite detection method for specific incubation products of the invention is western blotting. Firstly, a gel is mixed and cast, samples previously prepared are loaded onto the gel and fractionated by electrophoresis. The proteins present in the polyacrylamide gel are blotted onto a nitrocellulose membrane to which antibodies may be applied to detect the specific protein of interest. Western blotting is simply performed and advantageously when an exact determination of the concentration is dispensable.

A distinct number of specific antibodies against the protein markers of the invention is available. Antibodies are usually produced in mammal organisms when an immune response is caused by antigens being strange to the organism and having a molecular weight which exceeds 3.000 g/mol. Favorable host species for antibody production comprise goat, rabbit, and mouse. Further polyclonal and monoclonal antibodies can be selected originated from different species and fragments thereof. Popular techniques, such as the hybridoma technology, are well-known to the skilled artisan. The antibodies are applied as specific substances in the inventive method.

The antibodies can be immobilized onto a variety of solid supports, such as magnetic or chromatographic matrix particles, the surface of an assay plate {e.g. microtiter wells), pieces of a solid substrate material or membrane {e.g. plastic, nylon, paper) and the like. An assay strip can be prepared by coating the antibody or a plurality of antibodies in an array on a solid support. This strip can then be dipped into the test sample and processed quickly through washes and detection steps to generate a measurable signal, such as a colored spot.

The analysis can be carried out in a variety of physical formats. For example, the use of microtiter plates or automation could be used to facilitate the processing of large numbers of test samples. Alternatively, single sample formats could be developed to facilitate diagnosis or prognosis in a timely fashion. Useful physical formats comprise surfaces having a plurality of discrete, addressable locations for the detection of a plurality of different biomarkers. Such formats include protein microarrays or protein chips. In these embodiments, each discrete surface location may comprise antibodies to bind one or more protein markers for detection at each location. Surfaces may alternatively comprise one or more discrete particles (e.g. microparticles or nanoparticles) immobilized at discrete locations of a surface, where the microparticles comprise antibodies to immobilize one or more protein markers for detection. In one embodiment, a matrix is provided, in which each position represents a discrete binding site for a protein, and in which binding sites are present for all proteins p-p53 (Ser15), p21, p-H2A.X (Ser139), p-ATM (Ser1981), p-Chk1 (Ser345), p-ATR (Ser428), p-cdc2 (Thr14/Tyr15), Gadd45a and p-Chk2 (Thr68).

A signal from the direct or indirect label can be analyzed, for example, using a spectrophotometer to detect color from a chromogenic substrate, using a radiation counter to detect radiation, such as a gamma counter for detection of ¹²⁵I, or using a fluorometer to detect fluorescence in the presence of light of a certain wavelength. For detection of enzyme-linked antibodies, a quantitative analysis can be made using a spectrophotometer, such as an EMAX Microplate Reader (Molecular Devices; Menlo Park, CA) in accordance with the manufacturer's instructions. If desired, the assays of the present invention can be automated or performed robotically, and the signal from multiple samples can be detected simultaneously. In detail, the proteins can be detected by fluorescence which is recorded, for example, with a fluorescence laser microscope and a CCD camera, and the fluorescence intensity is analyzed with a computer. Optical images viewed and optionally recorded by a camera or other recording device (e.g. a photodiode and data storage device) are optionally further processed in any of the embodiments herein, e.g. by digitizing the image and storing and analyzing the image on a computer. A variety of commercially available peripheral equipment and software is available for digitizing, storing and analyzing a digitized video or digitized optical image. One conventional system carries light from the specimen field to a cooled charge-coupled device (CCD) camera, in common use in the art. A CCD camera includes an array of picture elements (pixels). The light from the specimen is imaged on the CCD. Particular pixels corresponding to regions of the specimen are sampled to obtain light intensity readings for each position. Multiple pixels are processed in parallel to increase speed. The apparatus and methods of the invention are easily used for viewing any sample, e.g. by fluorescent or dark field microscopic techniques.

In another embodiment of the screening method, the detection of mutagenic and/or pro-mutagenic activity can be additionally refined. For this purpose, the protein expression is determined by detecting several proteins selected from the group of p-p53 (Ser15), p21, p-H2A.X (Ser139), p-ATM (Ser1981), p-Chk1 (Ser345), p-ATR (Ser428), p-cdc2 (Thr14/Tyr15), Gadd45a and p-Chk2 (Thr68), and correlating an amount of signal, or change in signal, with the protein expression in the system. In other words, the expression levels of the active forms of the proteins correlate with an amount of an emitted physical signal, or change in an emitted physical signal. The cellular system of the invention is incubated with various concentrations of an identified (pro-)genotoxic compound. The amount of emitted signal, or change in signal, observed in the presence of the mutagenic compound is indicative of the change in protein expression experienced by the compound. The change can be then related to the concentration of the mutagen in the sample, i.e. the calibration curve enables the meter-reading of a matching concentration. Preferably, the calibration curve is based on the Lambert-Beer equation if using UV/VIS coloring or luminescence. Genotoxicity of compounds is diagnosed by comparing the concentration of the protein in the sample with known protein concentration levels of cells treated with mutagens and/or not. It shall be understood that the known concentrations are statistically proven, therefore representing a certain level or range, respectively. The direction and strength of protein expression have also been figured out by the differential expression analysis of the marker proteins of the invention such that a distinct up-regulation with a certain factor has been recognized. Any measured concentration, which differs from the protein concentration level of non-stimulated cells, indicates an abnormality of the tested cell sample, whereas a compound cannot be classified as mutagen at a protein concentration which is comparable to the concentration level of non-stimulated cells. It is preferred to measure concentrations, which are higher than the protein concentration level of non-stimulated cells, for detecting genotoxicity. Using this method, the inventors demonstrated sensitivity to submicromolar or even nanomolar concentrations. The calibration plot reveals that the method can be applied in a dynamic range that spans over a couple of magnitude.

Therefore, the method of the invention includes that a level of genotoxic and/or pro-genotoxic activity is screened by comparing the protein expression level in the test system with the protein expression level in the control system.
Although the biomarker panel of the invention exhibits a sensitivity that allows the use of only five marker proteins in the scope of the screening method, it is preferred to apply more than these marker proteins for detecting genotoxicity. The inventors have illustrated that analyzing multiple mutagen-responsive proteins increases screening stability and reduces error rates by covering a broader spectrum of genotoxic responses than low-plurality-protein reporter assays. In a preferred embodiment of the present invention, the expression levels of at least five, six, seven, eight or nine of said proteins are determined, more preferably at least five proteins, most preferably nine proteins. At least the proteins p-p53 (Ser15), p21, p-H2A.X (Ser139), p-ATM (Ser1981) and p-Chk1 (Ser345) are determined. In addition to the expression of said five proteins, the expression levels of one or more proteins selected from the group of p-ATR (Ser428), p-cdc2 (Thr14/Tyr15) and p-Chk2 (Thr68) can be determined, particularly selected from the group of p-ATR (Ser428), p-cdc2 (Thr14/Tyr15), Gadd45a and p-Chk2 (Thr68).

In another most preferred embodiment of the invention, the expression levels of at least the proteins p-p53 (Ser15), p21, p-H2A.X (Ser139), p-Chk1 (Ser345), p-ATR (Ser428), p-Chk2 (Thr68) and optionally MDM2 are determined. It is still another most preferred embodiment of the invention that the expression levels of at least the proteins p-p53 (Ser15), p-H2A.X (Ser139), p-Chk1 (Ser345) and p-Chk2 (Thr68) as well as total protein levels of p21 and optionally ATR and MDM2 are determined.

It is highly preferred that the expression levels of the proteins p-p53 (Ser15), p21, p-H2A.X (Ser139), p-ATM (Ser1981), p-Chk1 (Ser345), p-ATR (Ser428), p-cdc2 (Thr14/Tyr15), Gadd45a and p-Chk2 (Thr68) are determined.

It goes without saying that the expression of said respective preferred marker proteins in any preferred embodiment is compared with the protein expression in the control system.

If the system or the sample thereof is capable of expressing multiple proteins and an expression pattern of multiple proteins is compared with the expression pattern in the control system, the genotoxicity can be characterized compound-specifically. Particularly, the expression pattern is determined by a correlation of the multiple proteins and/or a magnitude of altered regulation. The screening method of this invention does not only evaluate the effect of chemical substances having genotoxic activity on cells to be evaluated, but can also indicate the details of this effect. By individually evaluating the expression level of categorized proteins, it is possible to distinguish how chemical compounds having genotoxic activity that affect the cells to be evaluated.

The invention also teaches an embodiment of the screening method, wherein the expression levels are determined in a biological sample withdrawn from a mammal, preferably a laboratory mammal, administered with one or more compounds to be screened, in comparison with a mammal showing non-genotoxic effects, wherein an increase in the expression levels indicates an increased likelihood that the compound has a therapeutic effect for a genotoxicity-susceptible pathological condition. With the therapeutic effect, the qualitative level is incorporated. A "therapeutic effect" relieves to some extent one or more symptoms of a disease or returns to normality, either partially or completely, one or more physiological or biochemical parameters associated with or causative of the disease or pathological conditions. In addition, the expression "therapeutically effective amount" denotes an amount which, compared with a corresponding subject who has not received this amount, has the following consequence: improved treatment, healing, prevention or elimination of a disease, syndrome, condition, complaint, disorder or side-effects or also the reduction in the advance of a disease, complaint or disorder. The expression "therapeutically effective amount" also encompasses the amounts which are effective for increasing normal physiological function. Testing of several compounds makes the selection of that compound possible that is best suited for the treatment of the mammal subject. The in-vivo dose rate of the chosen compound is advantageously pre-adjusted to the specific cells with regard to their in-vitro data. Therefore, the therapeutic efficacy is remarkably enhanced.
In another aspect of the invention, the least (pro-)genotoxic compound or a non-(pro-)genotoxic compound is identified, e.g. within a series of compounds, wherein the least increase or no increase in the expression levels indicates said compound, which is suitable for its intended use. The use could be therapeutic or non-therapeutic and is not limited to any particular purpose but complies with the intended use as known in the art (e.g. manual, paper, etc.). In a preferred aspect of the invention, a method for administering a therapeutic compound to a patient is provided, comprising performing the screening method of the invention with a series of compounds, identifying the compound having no genotoxic and pro-genotoxic activity, and administering said compound to a patient in need of the compound's intended use. It shall be understood that the method does not aim the identification of any use and/or the most active compound for said use but the elimination of adversary (pro-)genotoxic effects in the course of the intended use.
The invention also relates to a method for monitoring the likelihood of developing cancer, tumor, metastasis and/or disorder of angiogenesis, which are caused, mediated and/or propagated by deregulation of proliferation, differentiation and/or damage repair, in response to a treatment with a compound, wherein the expression levels of at least five proteins selected from the group of p-p53 (Ser15), p21, p-H2A.X (Ser139), p-ATM (Ser1981), p-Chk1 (Ser345), p-ATR (Ser428), p-cdc2 (Thr14/Tyr15), Gadd45a and p-Chk2 (Thr68) are determined in a biological sample withdrawn from a mammal in need of such treatment with said compound administered to said mammal, wherein an increase in the expression levels of at least five of said proteins indicates that said compound has genotoxic and/or pro-genotoxic activity and said likelihood is increased. The compound is preferably obtained by the screening method of the invention as set forth above. Thus, the prior teaching of the present specification concerning the screening method is valid and applicable without restrictions to method of monitoring if expedient.

The identification of the plurality of genes described above provides a powerful tool for assessing the progression of a state, condition or treatment. Specifically, a plurality of genes can be identified in a patient prior to an event, such as surgery, the onset of a therapeutic regime, or the completion of a therapeutic regime, to provide a base line result. This base-line can then be compared with the result obtained using identical methods either during or after such event. This information can be used for both diagnostic and prognostic purposes.

The inventive method of monitoring can be employed in human and veterinary medicine. The mammal is preferably a laboratory animal and/or a non-human organism. Herein, the compounds can be administered before or following an onset of disease once or several times acting as therapy. The terms "effective amount" or "effective dose" or "dose" are interchangeably used herein and denote an amount of the pharmaceutical compound having a prophylactically or therapeutically relevant effect on a disease or pathological conditions, i.e. which causes in a tissue, system, animal or human a biological or medical response which is sought or desired, for example, by a researcher or physician.

The aforementioned medical products of the inventive use are particularly used for the therapeutic treatment. Monitoring is considered as a kind of treatment, wherein the compounds are preferably administered in distinct intervals, e.g. in order to booster the response and eradicate the pathogens and/or symptoms of the genotoxicity-mediated disease completely. Either the identical compound or different compounds can be applied. The medicament can also be used to reduce the likelihood of developing a disease or even prevent the initiation of those diseases in advance that are associated with proliferation, differentiation and/or damage repair because of a genotoxic impact, or to treat the arising and continuing symptoms. In the meaning of the invention, prophylactic treatment is advisable if the subject possesses any preconditions for the aforementioned physiological or pathological conditions, such as a familial disposition, a genetic defect, or a previously passed disease. The diseases as concerned by the invention are preferably cancer, tumors, metastasis and/or disorders of angiogenesis.

The said compounds according to the invention can be used in their final non-salt form. On the other hand, the present invention also encompasses the use of these compounds in the form of their pharmaceutically acceptable salts, which can be derived from various organic and inorganic acids and bases by procedures known in the art. The expressions "pharmaceutically acceptable salt" and "physiologically acceptable salt", which are used interchangeable herein, in the present connection are taken to mean an active ingredient which comprises a compound according to the invention in the form of one of its salts, in particular if this salt form imparts improved pharmacokinetic properties on the active ingredient compared with the free form of the active ingredient or any other salt form of the active ingredient used earlier. The pharmaceutically acceptable salt form of the active ingredient can also provide this active ingredient for the first time with a desired pharmacokinetic property which it did not have earlier and can even have a positive influence on the pharmacodynamics of this active ingredient with respect to its therapeutic efficacy in the body.
Moreover, the invention relates to an in-vitro method for predicting the likelihood that a patient will suffer from a tumor in response to a therapeutic treatment with a drug, comprising the steps of (i) measuring in a biopsy sample from tissue or plasma of said patient expression levels of at least the five proteins p-p53 (Ser15), p21, p-H2A.X (Ser139), p-ATM (Ser1981), p-Chk1 (Ser345), (ii) exposing ex-vivo a tissue sample from tissue or plasma of said patient to said drug, and (iii) measuring in said exposed sample of step (ii) the expression levels of said proteins specified in step (i) along with calculating the differences in expression levels measured in steps (i) and (iii), wherein an increase in the expression levels of at least five of said proteins obtained in this step (iii) compared to step (i) indicates an that said drug has genotoxic and/or progenotoxic activity and said likelihood is increased.

Object of the invention is also the use of the at least five proteins p-p53 (Ser15), p21, p-H2A.X (Ser139), p-ATM (Ser1981), p-Chk1 as marker proteins for screening compounds with genotoxic and/or pro-genotoxic activity. The prior teaching of the present specification concerning the screening method is valid and applicable without restrictions to said uses if expedient.
It is still another object of the present invention to use substances specifically interacting with at least one marker protein for detecting genotoxic or pro-genotoxic activity. It is a preferred aspect of the present invention to use at least five antibodies, each with specific binding to a different protein selected from the group of p-p53 (Ser15), p21, p-H2A.X (Ser139), p-ATM (Ser1981), p-Chk1 (Ser345), for detecting genotoxic and/or pro-genotoxic activity. The antibodies are preferably directly labeled with isotopes, e.g. chromophores, luminphores or chromogens, or indirectly labeled with biotin to which a streptavidin complex may later bind. By assaying the presence or absence of the antibodies, one can detect the presence or absence of a marker protein of interest. The prior teaching of the present specification concerning the screening method is considered as valid and applicable without restrictions to said use of said anti-marker antibodies if expedient.

The invention may also be practiced as a use of a kit in the detection of genotoxic and/or pro-genotoxic activity comprising nine antibodies, each with specific binding to a different protein selected from the group of p-p53 (Ser15), p21, p-H2A.X (Ser139), p-ATM (Ser1981), p-Chk1 (Ser345), p-ATR (Ser428), p-cdc2 (Thr14/Tyr15), Gadd45a and p-Chk2 (Thr68). The kit is particularly designed to perform the inventive method for screening compounds with genotoxic and/or pro-genotoxic activity. The kit of the invention may include an article that comprises written instructions or directs the user to written instructions for how to practice the method of the invention. The prior teaching of the present specification concerning the screening method is considered as valid and applicable without restrictions to the kit if expedient.
In the scope of the present invention, a method for screening compounds with genotoxic activity, which applies unique protein expression patterns of up to nine proteins, is provided for the first time. The present invention teaches characteristic expression fingerprints of marker proteins that are associated with genotoxicity. The data support that mechanistic profiling in-vitro is a powerful tool compared to single endpoint detections to predict genotoxicity. Mechanistic profiling is of benefit during interpretation of such data, and mechanistic investigations are a powerful tool facilitating classification of genotoxic compounds. Furthermore, mechanistic data will improve chemical characterization and risk assessment for genotoxic compounds. Applying protein profiling to early screening during pharmaceutical development helps to rank different molecules and highlight compounds with genotoxic characteristics early in development, saving costs and animals by preventing follow-up testing in-vivo.
The nine putative marker proteins found can be used for screening unknown compounds, which effectively allows a specific and sensitive identification and characterization of their genotoxic and/or pro-genotoxic potential in the early phase of drug development. Genotoxic stress of the cellular system, which is able to functionally express up to nine marker proteins, leads to an increased expression and activation of these marker proteins compared to a control of this cellular system. The aforementioned proteins in combination have a synergistic effect, and therefore allow detecting genotoxins with a high performance. The analysis of the differential expressed proteins is particularly suitable for high throughput test systems. High Content Imaging (HCl)-based protein expression analyses can be favorably applied to genotoxicants with an unknown mode of action and predict their potential to exert genotoxic effects early in the drug discovery process. In addition to that, a cell-based model using HCl, has the advantage of generating data rapidly with small compound needs. The detection method as well as arising monitoring method of the invention can be performed in a simple, cost-efficient and reliable manner. Compounds having (pro-)genotoxic activity are screened with a sensitivity and/or specificity of at least 75%, more preferably at least 80%, most preferably at least 90%. Herein, "sensitivity" denotes the number of compounds that are correctly tested to be positive in relation to all positive tested compounds, and "specificity" denotes the number of compounds that are tested to be negative in relation to all negative compounds. This specific marker set can be combined with other genotoxicity endpoints like micronucleus assay to generate a highly predictive screening system for a broad range of potentially genotoxic compounds.

It is to be understood that this invention is not limited to the particular methods, specific substances, uses and arrays described herein, as such matter may vary. It is also to be understood that the terminology used herein is for the purpose of describing particular embodiments only and is not intended to limit the scope of the present invention, which is only defined by the appended claims. As used herein, including the appended claims, singular forms of words such as "a," "an," and "the" include their corresponding plural referents unless the context clearly dictates otherwise. Thus, e.g. reference to "a compound" includes a single or several different compounds, and reference to "a method" includes reference to equivalent steps and methods known to a person of ordinary skill in the art, and so forth. Unless otherwise defined, all technical and scientific terms used herein have the same meaning as commonly understood by a person of ordinary skill in the art to which this invention belongs.
The techniques that are essential according to the invention are described in detail in the specification. Other techniques which are not described in detail correspond to known standard methods that are well known to a person skilled in the art, or the techniques are described in more detail in cited references, patent applications or standard literature. Although methods and materials similar or equivalent to those described herein can be used in the practice or testing of the present invention, suitable examples are described below. The following examples are provided by way of illustration and not by way of limitation. Within the examples, standard reagents and buffers that are free from contaminating activities (whenever practical) are used. The example are particularly to be construed such that they are not limited to the explicitly demonstrated combinations of features, but the exemplified features may be unrestrictedly combined again if the technical problem of the invention is solved.
Table 1 lists the five proteins analyzed and the four supplementary markers with their post translational modifications, (alternative) protein names, (alternative) gene names and accession numbers, which are unique for each protein.
Table 2 lists the results for the nine protein markers (p-p53 (Ser15), p21, p-H2A.X (Ser139), p-ATM (Ser1981), p-Chk1 (Ser345), p-ATR (Ser428), p-cdc2 (Thr14/Tyr15), Gadd45a and p-Chk2 (Thr68)) tested with (pro-)genotoxins (cyclophosphamide, 7,12-dimethylbenzanthracene, aflatoxin B₁, 2-acetylaminofluorene, actinomycin D, methyl methanesulfonate, etoposide) and non-genotoxins (D-mannitol, phenfomin HCl, progesterone). Positive/negative results are displayed by the colors red/green, respectively.
Table 3 lists the group 1 chemicals ("in-vivo genotoxins which should be detected as positive in in-vitro mammalian cell genotoxicity tests") recommended by the European Center for the Validation of Alternative Methods (ECVAM) in order to judge the performance of in-vitro genotoxicity tests (Kirkland et al., 2008, Mutat. Res. 653, 99-108).
Table 4 lists the group 2 chemicals ("non-DNA-reactive chemicals, including non-genotoxic carcinogens, which should give negative results in in-vitro mammalian cell genotoxicity tests") recommended by the European Center for the Validation of Alternative Methods (ECVAM) in order to judge the performance of in-vitro genotoxicity tests (Kirkland et al., 2008, Mutat. Res. 653, 99-108).
Table 5 lists the group 3 chemicals ("Chemicals that should give negative results in in-vitro mammalian cell genotoxicity tests but have been reported to induce chromosomal aberrations or tk mutations in mouse lymphoma cells, often at high concentrations or at high levels of cytotoxicity.") recommended by the European Center for the Validation of Alternative Methods (ECVAM) in order to judge the performance of in-vitro genotoxicity tests (Kirkland et al., 2008, Mutat. Res. 653, 99-108).
Table 6 lists the group 1 chemicals ("in-vivo genotoxins which should be detected as positive in in-vitro mammalian cell genotoxicity tests") recommended by the ECVAM and their highest concentration tested limited by autofluorescence (AF), cytotoxicity based on previous experiments (Cyto) and precipitations (Prec) as well as the highest concentration <50% for the two cytotoxicity parameters selected cell count per valid field (SCC) and CMFDA cytoplasm average intensity (CMFDA).
Table 7 lists the group 2 chemicals ("non-DNA-reactive chemicals, including non-genotoxic carcinogens, that should give negative results in in-vitro mammalian cell genotoxicity tests") recommended by the ECVAM and their highest concentration tested limited by autofluorescence (AF), cytotoxicity based on previous experiments (Cyto) and precipitations (Prec) as well as the highest concentration <50% for the two cytotoxicity parameters selected cell count per valid field (SCC) and CMFDA cytoplasm average intensity (CMFDA).
Table 8 lists the group 3 chemicals ("Chemicals that should give negative results in in-vitro mammalian cell genotoxicity tests but have been reported to induce chromosomal aberrations or tk mutations in mouse lymphoma cells, often at high concentrations or at high levels of cytotoxicity.") recommended by the ECVAM and their highest concentration tested limited by autofluorescence (AF), cytotoxicity based on previous experiments (Cyto) and precipitations (Prec) as well as the highest concentration <50% for the two cytotoxicity parameters selected cell count per valid field (SCC) and CMFDA cytoplasm average intensity (CMFDA).
Table 9 lists the results for the five protein markers (p-p53 (Ser15), p21, p-H2A.X (Ser139), p-ATM (Ser1981), p-Chk1 (Ser345)) tested with the group 1 chemicals ("in-vivo genotoxins which should be detected as positive in in-vitro mammalian cell genotoxicity tests") recommended by the ECVAM. Positive/ negative results are displayed by the colors red/green, respectively.
Table 10 lists the results for the five protein markers (p-p53 (Ser15), p21, p-H2A.X(Ser139), p-ATM (Ser1981), p-Chk1 (Ser345)) tested with the group 2 chemicals ("non-DNA-reactive chemicals, including non-genotoxic carcinogens, that should give negative results in in-vitro mammalian cell genotoxicity tests") recommended by the ECVAM. Positive/negative results are displayed by the colors red/green, respectively.
Table 11 lists the results for the five protein markers (p-p53 (Ser15), p21, p-H2A.X(Ser139), p-ATM (Ser1981), p-Chk1 (Ser345)) tested with the group 3 chemicals ("Chemicals that should give negative results in in-vitro mammalian cell genotoxicity tests but have been reported to induce chromosomal aberrations or tk mutations in mouse lymphoma cells, often at high concentrations or at high levels of cytotoxicity.") recommended by the ECVAM. Positive/negative results are displayed by the colors red/green, respectively.
Table 12 lists the results of changes in phosphorylated p53 (Ser15) and total protein level of p21 in cell lysates using a MILLIPLEX MAP Magnetic Bead kit and Luminex system.

### EXAMPLE

### • Chemicals and cell culture media supplements

The compound set tested is recommended by the European Center for the Validation of Alternative Methods (ECVAM) in order to judge the performance of in-vitro genotoxicity tests: The two out of three groups of compounds which were already tested are listed (Table 3, 4 and 5) (Kirkland et al., 2008, Mutat. Res. 653, 99-108). All compounds tested were obtained at a minimum purity of 97%. Most of the compounds were ordered from Sigma-Aldrich (Taufkirchen, Germany) except Cyclophosphamide which was from Calbiochem (Darmstadt, Germany); 2-Acetylaminofluorene and Taxol were from Acros Organics (Geel, Belgium), Dimethylnitrosamine and Fluometuron were from Dr. Ehrenstorfer GmbH (Augsburg, Germany), 2-Amino-3methylimidazo[4,5-f]quinolone and 2-Amino-1-methyl-6-phenylimidazo[4,5-f]pyridine were from Apollo Scientific Ltd. (Bredbury, UK), N,N-Dicyclohexylthiourea and Amitrole were from TCI Europe (Zwijndrecht, Belgium) and Ephidrin sulfate was from LGC (Teddington, UK). DMEM/F12, gentamicin, and sodium pyruvate were purchased from Invitrogen Corp. (Karlsruhe, Germany). Foetal bovine serum (FBS) was obtained from HyClone (Order No. SV30160.03, Lot No. RSJ30856, HyClone UK Ltd., Cramlington, UK). B-naphthoflavone/ phenobarbital-induced rat liver S9 (Order No. R1081.S9, Lot No. 0710507) was ordered from Tebu-bio (Offenbach, Germany). Trypsin, β-nicotinamide adenine dinucleotide 2'-phosphate reduced tetrasodium salt hydrate (NADPH) and penicillin/streptomycin solution were obtained from Sigma-Aldrich (Taufkirchen, Germany). Magnesium chloride, potassium chloride, sodium phosphate monobasic monohydrate and sodium phosphate dibasic heptahydrate were purchased from Merck KGaA (Darmstadt, Germany).

### • Cell culture

HepG2 cells (Order No. HB-8065, Lot. 58483209, ATCC, Manassas, USA) were cultured in DMEM/F12 with L-Glutamine and 15 mM Hepes supplemented with 10% (v/v) FBS, 1% (v/v) penicillin (10 kU/ml)/ streptomycin (10 mg/ml) solution, 0.1% (v/v) gentamicin (50 mg/ml), and 1 mM sodium pyruvate at 37°C and 5% CO₂ in culture flasks. Depending on the experiment, an appropriate number of cells were seeded onto plates after detaching of the cells using trypsin and cells were cultured at 37°C and 5% CO₂ for 24 h prior to treatment. All experiments were performed three times with cells of passages 4-20.

### Testing of nine putative marker proteins

### • Cell treatment and dose selection

Cells were seeded onto black Poly-D-Lysin coated 96-well-plates (BD Biocoat, Franklin Lakes, USA) (0.02 x 10⁶ cells/well) and left 24 h for adherence and then treated with test compounds. For each compound, seven concentrations were tested with 2-fold serial dilutions. DMSO 1 % (v/v) served as vehicle control for the experiments. The treatment procedure was different for direct-genotoxic and pro-genotoxic compounds. The treatment for direct genotoxic compounds was ongoing and was repeated daily over 48 h. Pro-genotoxic compounds were incubated for 6 h together with metabolic activation system (to limit the cytotoxic effect of the S9-mixture). After this period, cells were washed with cell media followed by 18-repeated daily over 48 h. The metabolic activation system used consisted of the following components and concentrations: 8 mM MgCl₂, 32.8 mM KCI, 12 mM NADPH, 124 mM phosphate buffer and 2500 pmol/ml cytochrome P450 (CYP) content in the pre-mixture corresponding to 2.4 mM MgCl₂, 9.8 mM KCI, 3.6 mM NADPH, 37.2 mM phosphate buffer, and 750 pmol/ml CYP as final concentrations after dilution 1:3.33 with cell culture media.

### • Immunofluorescene staining

### - Staining protocol for cytotoxicity parameters:

After treatment, cells were rinsed with PBS (Gibco Invitrogen, Karlsruhe, Germany), and then stained with 10 µM CellTracker Green 5-Chloromethylfluorescein Diacetate (CMFDA) (Invitrogen, Karlsruhe, Germany) in cell culture media. CMFDA is per se non-fluorescent and is hydrolyzed by esterase in viable cells to a fluorescent-dye. The reaction with thiol groups leads to a cell-impermeable fluorescent-dye-adduct. The esterase activity can be used as an indicator for viability of cells (Papadopoulos et al., 1994, J Immunol Methods 177, 101-111). After an incubation period of 30 min at 37°C, cells were washed with PBS and then fixed with 3.7% formaldehyde (Sigma-Aldrich, Taufkirchen, Germany) in PBS for 20 min. Afterwards, two additional washing steps with 0.05% Tween 20 followed, then cells were washed with PBS and finally PBS was added and cells were measured.

### - Staining protocol for putative marker proteins:

After treatment, cells were rinsed with PBS (Gibco Invitrogen, Karlsruhe, Germany), and then fixed with 3.7% formaldehyde (Sigma-Aldrich, Taufkirchen, Germany) in PBS for 20 min. After washing twice with PBS, cells were blocked and permeabilized with 7.5% goat serum (Sigma-Aldrich, Taufkirchen, Germany) and 0.3% Triton X-100 (Sigma-Aldrich, Taufkirchen, Germany) in PBS for 30 min. Each primary antibodies were diluted separately to final concentration of 10 µg/ml anti-Gadd45α rabbit monoclonal antibody IgG (Cell Signaling, Danvers, USA), 10 µg/ml anti-p21 rabbit monoclonal antibody IgG (Cell Signaling, Danvers, USA); 5 µg/ml anti-p-ATM (Ser1981) rabbit monoclonal antibody IgG (Cell Signaling, Danvers, USA); 10 µg/ml anti-p-ATR (Ser428) rabbit monoclonal antibody IgG (Cell Signaling, Danvers, USA); 4 µg/ml anti-p-cdc2 (Tyr15/Thr14) rabbit polyclonal antibody IgG (Santa Cruz Biotechnology, Inc., Santa Cruz, USA); 20 µg/ml anti-p-Chk1 (Ser345) rabbit monoclonal antibody IgG (Cell Signaling, Danvers, USA); 20 µg/ml anti-p-Chk2 (Thr68) rabbit monoclonal antibody IgG (Cell Signaling, Danvers, USA); 2.5 µg/ml anti-p-Histone H2A.X (Ser139) rabbit monoclonal antibody IgG1 (Cell Signaling, Danvers, USA) or 2 µg/ml anti-p-p53 (Ser15) rabbit monoclonal antibody IgG (Invitrogen, Karlsruhe, Germany) in Blocking/Permeabilization solution were added afterwards. After incubation for 16-18 h, cells were washed twice with 0.05% Tween 20 (Calbiochem, Darmstadt, Germany) in PBS and the secondary antibody/16 µM Hoechst staining solution in Blocking/Permeabilization solution was added. Secondary antibody (Alexa Fluor 488 goat anti-rabbit IgG, Invitrogen, Karlsruhe, Germany) was diluted to a final concentration 2 µg/ml. After an incubation of 1 h, followed by two additional washing steps with 0.05% Tween 20, cells were washed with PBS and finally PBS was added and cells were measured.

### • Image acquisition and analysis for cytotoxicity parameters:

Image acquisition was performed on the ArrayScan VTI HCS Reader (Cellomics, Pittsburgh, USA) using a 10x objective. To detect enough cells, 20 images starting at the middle of each well were gathered.

The filter for were chosen according to the excitation/ emission wavelengths for each dye:
- Protocol for cytotoxicity parameters:
   Channel 1: 365 ± 25 and 515 ± 10 nm (XF93- Hoechst) for Hoescht 33342
   Channel 2: 475 ± 20 and 515 ± 10 nm (XF93- FITC) for CMFDA

### • Image acquisition and analysis for putative marker proteins:

Image acquisition was performed on the ArrayScan VTI HCS Reader (Cellomics, Pittsburgh, USA) using a 20x objective. To detect enough cells, 20 images starting at the middle of each well were gathered.

The filter for were chosen according to the excitation/ emission wavelengths for each dye:
- Protocol for putative marker proteins:
   Channel 1: 365 ± 25 and 515 ± 10 nm (XF93- Hoechst) for Hoescht 33342
   Channel 2: 475 ± 20 and 515 ± 10 nm (XF93- FITC) for Alexa Fluor 488 goat anti-rabbit

The analysis of the images was performed with the software iDev and the Bioapplication Version 4 (Cellomics, Pittsburgh, USA). The stained nuclei were used for localization of the nucleus and the cytoplasm (ring around the nucleus).

The following readout parameters were generated:
- Protocol for cytotoxicity parameters:
   Channel 1: selected cell count per valid field
   Channel 2: CMFDA cytoplasm average intensity
- Protocol for putative marker proteins:
   Channel 2: protein nucleus average intensity

### • Data analysis and interpretation

Fold-regulations of treated samples against the vehicle control were calculated for each compound and concentration. Statistical significance (p-value < 0.05) was determined using the Student's t-test. The thresholds for each parameter were chosen based on the control value and addition of three times the standard deviation compared to the control value. For p-p53 (Ser15), p-H2A.X (Ser139) and p-ATM (Ser1981) the thresholds were set to 1.5, for p21, p-Chk1 (Ser345), p-ATR (Ser428), p-cdc2 (Tyr15/Thr14) and Gadd45 α to 1.6 and p-Chk2 (Thr68) to 1.9. Concentrations with precipitation or autofluorescence were excluded as well as concentrations which showed at least in one of the two cytotoxicity parameters (selected cell count per valid field and CMFDA intensity in the cytoplasm) a cytotoxic effect ≥50%.

### Testing of five marker proteins

### • Cell treatment and dose selection

Cells were seeded onto black Poly-D-Lysin coated 96-well-plates (BD Biocoat, Franklin Lakes, USA) (0.02 x 10⁶ cells/well) and left 24 h for adherence and then treated with test compounds at a maximum concentration of 1 mM which is recommended by the ICH S2(R1) guideline (EMA, 2011). For each compound, nine concentrations were tested with 2-fold serial dilutions. DMSO 1 % (v/v) served as vehicle control for the experiments. The treatment procedure was different for direct-genotoxic and pro-genotoxic compounds. The treatment for direct genotoxic compounds was ongoing and was repeated daily over 48 h. Pro-genotoxic compounds were incubated for 6 h together with metabolic activation system (to limit the cytotoxic effect of the S9-mixture). After this period, cells were washed with cell media followed by 18-repeated daily over 48 h. The metabolic activation system used consisted of the following components and concentrations: 8 mM MgCl₂, 32.8 mM KCI, 12 mM NADPH, 124 mM phosphate buffer and 2500 pmol/ml cytochrome P450 (CYP) content in the pre-mixture corresponding to 2.4 mM MgCl₂, 9.8 mM KCl, 3.6 mM NADPH, 37.2 mM phosphate buffer, and 750 pmol/ml CYP as final concentrations after dilution 1:3.33 with cell culture media.

### • Immunofluorescene staining

Two different staining procedures were performed for each compound with and without metabolic activation system:

### - Staining protocol 1:

After treatment, cells were rinsed with PBS (Gibco Invitrogen, Karlsruhe, Germany), and then fixed with 3.7% formaldehyde (Sigma-Aldrich, Taufkirchen, Germany) in PBS for 20 min. After an additional washing step with PBS, cells were blocked and permeabilized with 2% donkey serum (Millipore, Schwalbach, Germany) and 0.25% Triton X-100 (Sigma-Aldrich, Taufkirchen, Germany) in PBS for 30 min. Primary antibodies were diluted to final concentrations of 2 µg/ml anti-p-p53 (Ser15) rabbit monoclonal antibody IgG (Invitrogen, Karlsruhe, Germany), 0.8 µg/ml anti-p21 goat polyclonal antibody IgG, (R&D systems, Minneapolis, USA); 4 µg/ml anti-p-Histone H2A.X (Ser139) mouse monoclonal antibody IgG1 (Millipore, Schwalbach, Germany) in Blocking/Permeabilization solution were added afterwards. After incubation for 16-18 h, cells were washed with 0.05% Tween 20 (Calbiochem, Darmstadt, Germany) in PBS and the secondary antibody/16 µM Hoechst staining solution in Blocking/Permeabilization solution was added. Secondary antibodies were diluted 1:400 (Alexa Fluor 488 donkey anti-rabbit, Alexa Fluor 555 donkey anti-goat, Alexa Fluor 647 donkey anti-mouse, Invitrogen, Karlsruhe, Germany). After an incubation of 1 h, followed by two additional washing steps with 0.05% Tween 20, cells were washed with PBS and finally PBS was added and cells were measured.

### - Staining protocol 2:

After treatment, cells were rinsed with PBS (Gibco Invitrogen, Karlsruhe, Germany), and then stained with 10 µM CellTracker Green 5-Chloromethylfluorescein Diacetate (CMFDA) (Invitrogen, Karlsruhe, Germany) in cell culture media for 30 min. CMFDA is per se non-fluorescent and is hydrolyzed by esterase in viable cells to a fluorescent-dye. The reaction with thiol groups leads to a cell-impermeable fluorescent-dye-adduct. The esterase activity can be used as an indicator for viability of cells (Papadopoulos et al., 1994, J Immunol Methods 177, 101-11). Afterwards, cells were washed with PBS and fixed with 3.7% Formaldehyde (Sigma-Aldrich, Taufkirchen, Germany) in PBS for 20 min. After that, cells were washed with PBS and blocked and permeabilized with 2% donkey serum (Millipore, Schwalbach, Germany) and 0.25% Triton X-100 (Sigma-Aldrich, Taufkirchen, Germany) in PBS for 30 min. Primary antibodies were diluted in Blocking/Permeabilization solution to final concentrations of 4 µg/ml anti-p-Chk1 (Ser345) goat polyclonal antibody IgG (santa cruz, Santa Cruz, USA), 4 µg/ml anti-p-ATM (Ser1981) mouse monoclonal antibody IgG1K (Millipore, Schwalbach, Germany) and added to the cells. After incubation for 16-18 h, cells were washed with 0.05% Tween 20 (Calbiochem, Darmstadt, Germany) in PBS, and the secondary antibody/ 16 µM Hoechst staining solution Blocking/ Permeabilization solution was added. Secondary antibodies were diluted 1:400 (Alexa Fluor 555 donkey anti-goat, Alexa Fluor 647 donkey anti-mouse, Invitrogen, Karlsruhe, Germany). After an incubation of 1 h, followed by two additional washing steps with 0.05% Tween 20, cells were washed with PBS and finally PBS was added and cells were measured.

### • Image acquisition and analysis

Image acquisition was performed on the ArrayScan VTI HCS Reader (Cellomics, Pittsburgh, USA) using a 20x objective. To detect enough cells, 50 images starting at the middle of each well were gathered.

The filter for were chosen according to the excitation/ emission wavelengths for each dye:
- Protocol 1:
   Channel 1:365 ± 25 and 515 ± 10 nm (XF93- Hoechst) for Hoescht 33342
   Channel 2: 475 ± 20 and 515 ± 10 nm (XF93- FITC) for Alexa Fluor 488 donkey anti-rabbit
   Channel 3: 549 ± 4 and 600 ± 12.5 nm (XF93-TRITC) for Alexa Fluor 555 donkey anti-goat
   Channel 4: 655 ± 15 and 730 ± 25 nm (XF110-Cy5 (sensitive)) for Alexa Fluor 647 donkey anti-mouse
- Protocol 2:
   Channel 1:365 ± 25 and 515 ± 10 nm (XF93- Hoechst) for Hoescht 33342
   Channel 2: 475 ± 20 and 515 ± 10 nm (XF93- FITC) for CMFDA
   Channel 3: 549 ± 4 and 600 ± 12.5 nm (XF93-TRITC) for Alexa Fluor 555 donkey anti-goat
   Channel 4: 655 ± 15 and 730 ± 25 nm (XF110-Cy5 (sensitive)) for Alexa Fluor 647 donkey anti-mouse.

The analysis of the images was performed with the software iDev and the Bioapplication Version 4 (Cellomics, Pittsburgh, USA). The stained nuclei in both assays were used for localization of the nucleus and the cytoplasm (ring around the nucleus).

The following readout parameters were generated for each well:
- Protocol 1:
   Channel 2: p-p53 (Ser15) nucleus average intensity
   Channel 3: p21 nucleus average intensity
   Channel 4: p-H2A.X (Ser139) nucleus average intensity
- Protocol 2:
   Channel 1: selected cell count per valid field
   Channel 2: CMFDA cytoplasm average intensity
   Channel 3: p-Chk1 (Ser345) nucleus average intensity
   Channel 4: p-ATM (Ser1981) nucleus average intensity

### • Data analysis and interpretation

Fold-regulations of treated samples against the vehicle control were calculated for each compound and concentration. Statistical significance (p-value < 0.05) was determined using the Student's t-test. The thresholds for each parameter were chosen based on the control value and addition of three times the standard deviation compared to the control value. For p-Chk1 (Ser345) and p-H2A.X (Ser139), the thresholds were set to 2.1 for p21, p-ATM (Ser1981) to 1.8 and p-p53 (Ser15) to 1.9. If at least one of the five proteins showed a fold-change above this threshold, the compound was set as positive. Otherwise the compound was set as negative. Concentrations with precipitation or immunofluorescence were excluded as well as concentrations which showed at least in one of the two cytotoxicity parameters (selected cell count per valid field and CMFDA intensity in the cytoplasm) a cytotoxic effect ≥ 50%.

The study aimed to develop a novel, specific and sensitive high content imaging-based test system for mutagens and promutagens in-vitro using HepG2 cells. Due to their limited metabolic capacity, a combined system of HepG2 cells and a metabolic activation system (MAS - rat liver S9) was established for promutagen testing. Up to nine different proteins involved in DNA-damage response served as putative markers for compound-induced genotoxicity. The protein expression- and activation changes were quantified 48h after treatment with (pro-)genotoxins (Cyclophosphamide, 7,12-Dimethylbenzanthracene, Aflatoxin B₁, 2-Acetylaminofluorene, Actinomycin D, Methyl methanesulfonate, Etoposide) and non-genotoxins (D-mannitol, Phenfomin HCl, Progesterone) using the HCl technology.

The best classification was achieved using five out of nine putative marker proteins. The five most predictive markers out of the aforementioned nine markers (p-p53 (Ser15), p21, p-H2A.X (Ser139), p-ATM (Ser1981), p-Chk1 (Ser345), p-ATR (Ser428), p-cdc2 (Thr14/ Tyr15), Gadd45a, p-Chk2 (Thr68)) were selected. While the experiments were predicated on the five markers p-p53 (Ser15), p21, p-H2A.X(Ser139), p-ATM (Ser1981), p-Chk1 (Ser345), an additional analysis of p-ATR (Ser428), p-cdc2 (Thr14/Tyr15), Gadd45a, p-Chk2 (Thr68) allowed a more detailed characterization of the compound's mode of action, and they were used as supplementary markers.

The analysis of the nine putative markers p-p53 (Ser15), p21, p-H2A.X (Ser139), p-ATM (Ser1981), p-Chk1 (Ser345), p-ATR (Ser428), p-cdc2 (Thr14/Tyr15), Gadd45a and p-Chk2 (Thr68) was quantified 48 h after treatment with (pro-)genotoxins (Cyclophosphamide, 7,12-Dimethylbenzanthracene, Aflatoxin B₁, 2-Acetylaminofluorene, Actinomycin D, Methyl methanesulfonate, Etoposide) and non-genotoxins (D-mannitol, Phenfomin HCl, Progesterone) using the HCl technology. The best classification was achieved using the five markers (p-p53 (Ser15), p21, p-H2A.X (Ser139), p-ATM (Ser1981) and p-Chk1 (Ser345)) out of nine putative marker proteins (Table 2).

The analysis of the group 1 chemicals ("in-vivo genotoxins which should be detected as positive in in-vitro mammalian cell genotoxicity tests") recommended by the ECVAM led to four false negative compounds. Ethylnitrosourea, dimethylnitrosamine, 2,4-diaminotoluene and cadmiumchlorid conducted no positive result for any of the protein markers tested. Therefore, the sensitivity of this test system with 18 compounds classified as positive out of 20 in-vivo genotoxins tested is 80% (Table 6 and 9).

The group 2 chemicals ("non-DNA-reactive chemicals, including non-genotoxic carcinogens, which should give negative results in in-vitro mammalian cell genotoxicity tests") led to two false positive tested compounds. Progesterone and Phenanthracene both gave a positive result for p-ATM (Ser1981) resulting in two false positive tested compounds out of 23 tested compounds. As a consequence, a specificity of 91.3% could be calculated (Table 7 and 10).

The group 3 chemicals ("non-DNA-reactive chemicals, including non-genotoxic carcinogens, metabolic poisons and others that should give negative results in-vitro in mammalian cell genotoxicity tests, but have been reported to induce chromosomal abberationas or tk mutations in mouse lymphoma cells, often at concentrations or at high levels of cytotoxicity") led to four false positive tested compounds. Propyl gallate, p-nitrophenol, Eugenol and 2,4-dichlorophenol gave positive results resulting in four false positive tested compounds out of 19 tested compounds. As a consequence, a specificity of 78.9% could be calculated (Table 8 and 11).

These five proteins were potential candidates for the identification of potential genotoxicants early in the drug discovery process.

### • Luminex analysis

A Magnetic Bead kit (Milliplex MAP) was used to detect changes in phosphorylated p53 (Ser15) as well as total protein level of p21 in cell lysates using the Luminex system.

MILLIPLEX MAP is based on the Luminex xMAP technology. Luminex uses proprietary techniques to internally color-code microspheres with two fluorescent dyes. Through precise concentrations of these dyes, 100 distinctly colored bead sets can be created, each of which is coated with a specific capture antibody. After an analyte from a test sample is captured by the bead, a biotinylated detection antibody is introduced. The reaction mixture is then incubated with Streptavidin-Phycoerythrin (SAPE) conjugate, the reporter molecule, to complete the reaction on the surface of each microsphere. The microspheres are illuminated, and the internal dyes fluoresce, marking the microsphere set(s) used in a particular assay. A second illumination source excites PE, the fluorescent dye on the reporter molecule. Finally, high-speed digital-signal processors identify each individual microsphere and quantify the result of its bioassay based on fluorescent reporter signals.

The performance of the assay was done as described below and reagents were used from the MILLIPLEX MAP kits (EMD Millipore Cat. # 46-662 and 46-621).

### - Cell lysis protocol

Adherent or non-adherent cells grown in sterile 96-well tissue culture grade plates could be treated, washed and lysed in the same plate, but needed to be filtered in a separate 96-well filter plate. The protocol steps were as follows: For non-adherent cells, the tissue culture plate was centrifuged for 2 minutes at 500 x g to pellet cells. If adherent cells were used, it was started with the next step. The media was removed via aspiration, and 100 µL ice-cold PBS or TBS were added. For non-adherent cells, the first step was repeated. The wash was removed via aspiration. It was added 35 µL/well ice-cold 1X MILLIPLEX MAP Lysis Buffer containing phosphatase inhibitors (including 1 mM sodium orthovanadate (Na₃VO₄)) and freshly prepared protease inhibitors. The plate was placed on an orbital shaker (600-800 rpm) for 10-15 minutes at 4°C. The lysate was stored at -70°C until ready for use.

### - Immunoassay protocol

The filtered lysates were diluted at least 1:1 in MILLIPLEX® MAP Assay Buffer. The suggested working range of protein concentration for the assay was 1 to 25 µg of total protein/well (25 µL/ well at 40 to 1000 µg/mL). 50 µL of Assay Buffer were added into each well of the plate and covered and mixed on a plate shaker for 10 minutes at room temperature (20-25°C). The Assay Buffer was decanted, and the residual amount was removed from all wells by inverting the plate and tapping it smartly onto absorbent towels several times. The 1X bead suspension was vortexed for 10 seconds, and 25 µL of 1X bead suspension were added to each well. 25 µL of Assay Buffer, reconstituted control cell lysates and sample lysates were added to appropriate wells and incubated overnight (16-20 hours) at 2-8°C on a plate shaker (600-800 rpm) protected from light. A handheld magnetic separation block was attached to the plate, and 60 seconds were allowed for beads to settle before the samples and controls were decanted. The plate was removed from the magnetic separation block, and washed with 100 µL Assay Buffer per well. It was repeated for a total of two washes. 25 µL/well of 1X MILLIPLEX MAP Detection Antibody were added. The plate was sealed, covered with lid and incubated with agitation on a plate shaker for 1 hour at room temperature (20-25°C). The Magnetic Separation Block was attached, and it was waited for 60 seconds before the Detection Antibody was decanted. 25 µL of 1X MILLIPLEX MAP Streptavidin-Phycoerythrin (SAPE) were added. The plate was sealed, covered with lid and incubated with agitation on a plate shaker for 15 minutes at room temperature (20-25°C). 25 µL of MILLIPLEX MAP Amplification Buffer were added to each well. The plate was sealed, covered with lid and incubated with agitation on a plate shaker for 15 minutes at room temperature (20-25°C). The Magnetic Separation Block was attached, and it was waited for 60 seconds before the SAPE /Amplification buffer was decanted. The beads were suspended in 150 µL of MILLIPLEX MAP Assay Buffer, and mixed on plate shaker for 5 minutes before analysis using the Luminex system.

### - Data analysis and interpretation

Fold-regulations of treated samples against the vehicle control were calculated for each compound and concentration. The thresholds for each parameter were chosen based on the control value and addition of three times the standard deviation compared to the control value. For p-p53 (Ser 15) and p21 the threshold was set to 1.5. If at least one of the proteins showed a fold-change above this threshold, the compound was set as positive. Otherwise the compound was set as negative.

The results of the MILLIPLEX MAP Magnetic Bead kit, which was used to detect changes in phosphorylated p53 (Ser15) as well as total protein level of p21 in cell lysates using the Luminex system are shown in Table 12. The genotoxic compounds Methylmethane sulfonate and Etoposide as well as non-genotoxic D-mannitol were tested.

**Table 1**

| **Protein** (with post translational modification) | **Protein name** (alternative name) | **Gene name** (alternative name) | **Accession number** |
|---|---|---|---|
| p-p53 (Ser15) | Cellular tumor antigen p53, (Alternative name(s): Antigen NY-CO-13 Phosphoprotein p53 Tumor suppressor p53) | TP53 (P53) | P04637 |
| p21 | Cyclin-dependent kinase inhibitor 1, (CDK-interacting protein 1 Melanoma differentiation-associated protein 6, p21) | CDKN1A, (CAP20, CDKN1, CIP1, MDA6, PIC1, SDI1, WAF1) | P38936 |
| H2A.X (Ser139) | Histone H2A.X | H2AFX, (H2AX) | P16104 |
| p-Chk1 (Ser345) | Serine/threonine-protein kinase Chk1, (CHK1 checkpoint homolog Cell cycle checkpoint kinase Checkpoint kinase-1) | CHEK1, (CHK1) | 014757 |
| p-ATM(Ser1981) | Serine-protein kinase ATM, (Ataxia telangiectasia mutated) | ATM | Q13315 |

| **Supplementary marker** | | | |
|---|---|---|---|
| p-ATR (Ser428) | Serine/threonine-protein kinase ATR, (Ataxia telangiectasia and Rad3-related protein FRAP-related protein 1) | ATR, (FRP1) | Q13535 |
| p-cdc2 (Thr14/ Tyr15) | Cyclin-dependent kinase 1, (Cell division control protein 2 homolog Cell division protein kinase 1 p34 protein kinase) | CDK1, (CDC2, CDC28A, CDKN1, P34CDC2) | P06493 |
| Gadd45a | Growth arrest and DNA damage-inducible protein GADD45 alpha, (DNA damage-inducible transcript 1 protein) | GADD45A, (DDIT1, GADD45) | P24522 |
| p-Chk2 (Thr68) | Serine/threonine-protein kinase Chk2, (CHK2 checkpoint homolog Cds1 homolog, Checkpoint kinase 2) | CHEK2, (CDS1, CHK2, RAD53) | 096017 |

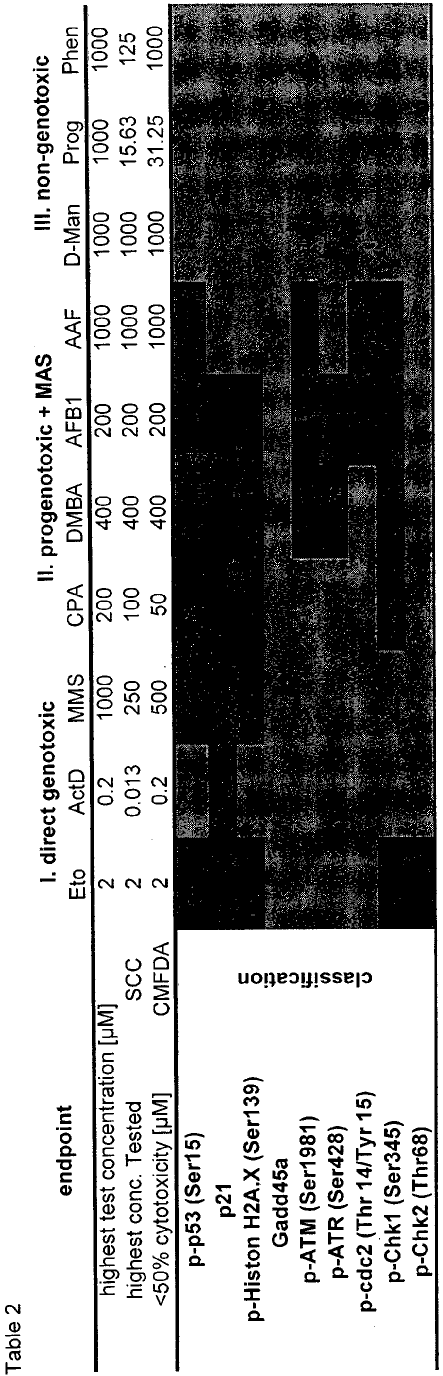

**Table 3**

| **compound** | **abbreviation** | **CAS number** |
|---|---|---|
| **I. Ames-positive in vivo genotoxins** | | |
| (i) 06 and N7 alkylators | | |
| Cyclophosphamide | CPA | 6055-19-2 |
| Ethylnitrosourea | ENU | 759-73-9 |
| Methylmethane sulfonate | MMS | 66-27-3 |
| | | |

| (ii) Polycyclic aromatic hydrocarbons | | |
|---|---|---|
| Benzo[a]pyrene | BaP | 50-32-8 |
| 7,12-Dimethylbenz[a]anthracene | DMBA | 57-97-6 |
| | | |

| (iii) Aromatic amines | | |
|---|---|---|
| Dimethylnitrosamine | DMN | 62-75-9 |
| 2-Acetylaminofluorene | AAF | 53-96-3 |
| 2,4-diaminotoluene | DAT | 95-80-7 |
| IQ (2-Amino-3methylimidazo[4,5-*f*]quinoline) | IQ | 76180-96-6 |
| PhIP.HCl (2-Amino-1-methyl-6-phenylimidazo[4,5-*f*]pyridine) | PhIP | 105650-23-5 |
| | | |

| (iv) Others | | |
|---|---|---|
| Aflatoxin B1 | AFB1 | 1162-65-8 |
| Cadmium chloride | CC | 10108-64-2 |
| Cisplatin | CP | 15663-27-1 |
| p-chloroaniline | CA | 106-47-8 |
| | | |

| **II. In vivo genotoxins negative or equivocal in Ames** | | |
|---|---|---|
| Etoposide | Eto | 33419-42-0 |
| Hydroquinone | HQ | 123-31-9 |
| Azidothymidine | AZT | 30516-87-1 |
| Sodium arsenite | SA | 7784-46-5 |
| Taxol | T | 33069-62-4 |
| Chloramphenicol | CIA | 56-75-7 |

**Table 4**

| **compound** | **abbreviation** | **CAS number** |
|---|---|---|
| **(i) Non-carcinogens with negative in vivo genotoxicity data** | | |
| Ampicillin trihydrate | AmpT | 7177-48-2 |
| D-mannitol | D-Man | 69-65-8 |
| | | |

| **(ii) Non-carcinogens with no in vivo genotoxicity data** | | |
|---|---|---|
| Phenformin HCl | Phen | 834-28-6 |
| n-butyl chloride | n-BCl | 109-69-3 |
| (2-chloroethyl)trimethyl-ammonium chloride | TAC | 999-81-5 |
| Cyclohexanone | CyH | 108-94-1 |
| N,N-dicyclohexyl thiourea | DCHT | 1212-29-9 |
| Trisodium EDTA trihydrate | TET | 150-38-9 |
| Ephidrine sulphate | EpS | 134-72-5 |
| Erythromycin stearate | EyS | 643-22-1 |
| Fluometron | FM | 2164-17-2 |
| Phenanthrene | PhA | 85-01-8 |
| | | |

| **(iii) Non-genotoxic carcinogens** | | |
|---|---|---|
| D-limonene | DL | 5989-27-5 |
| Di-(2-ethylhexyl)phthalate | DEPh | 117-81-7 |
| Am itrole | AT | 61-82-5 |
| Tert-butyl alcohol | TBA | 75-65-0 |
| Diethanolamine | DA | 111-42-2 |
| Melamine | Mel | 108-78-1 |
| Methyl carbamate | MetC | 598-55-0 |
| Progesterone | Pro | 57-83-0 |
| Pyridine | Pyr | 110-86-1 |
| Tris(2-ethylhexyl)phosphate | TEP | 78-42-2 |
| Hexachloroethane | HCE | 67-72-1 |

**Table 5**

| **(i) Non-carcinogens that are negative or equivocal for genotoxicity in vivo** | | |
|---|---|---|
| D,L-menthol | Men | 15356-70-4 |
| Phthalic anhydride | PhAh | 85-44-9 |
| Tertiary-butylhydroquinone | TBHQ | 1948-33-0 |
| *o*-Anthranilic acid | AA | 118-92-3 |
| 1,3-Dihydroxybenzene (resorcinol) | DB | 108-46-3 |
| 2-Ethyl-1,3-hexanediol | EH | 94-96-2 |
| Sulfisoxazole | SO | 127-69-5 |
| | | |
| **(ii) Non-carcinogens with no in vivo genotoxicity data** | | |
| Ethionamide | EtAm | 536-33-4 |
| Curcumin | Cu | 458-37-7 |
| Benzyl alcohol | BA | 100-51-6 |
| Urea | U | 57-13-6 |
| | | |

| **(iii) Non-genotoxic carcinogens or carcinogenic by irrelevant (for humans) mechanism** | | |
|---|---|---|
| Sodium saccharin | Ssa | 128-44-9 |
| | | |

| **(iv) Supplementary list (prediction of in vitro genotoxicity results less clear)** | | |
|---|---|---|
| Propyl gallate | PG | 121-79-9 |
| *p*-Nitrophenol | NPh | 100-02-7 |
| Sodium xylene sulfonate | SXS | 1300-72-7 |
| Ethyl acrylate | EtAc | 140-88-5 |
| Eugenol | Eu | 97-53-0 |
| Isobutyraldehyde | IBA | 78-84-2 |
| 2,4-Dichlorophenol | DP | 120-83-2 |

**Table 6**

| | | | **SCC** | | **CMFDA** | |
|---|---|---|---|---|---|---|
| | | | **-S9** | **+S9** | **-S9** | **+S9** |
| **I. Ames-positive in vivo genotoxins** | highest conc. | | highest conc. <50% | | | |
| (i) 06 and N7 alkylators | mM | limit | cytotox. over control [µM] | | | |
| Cyclophosphamide | 1.00 | mM | 1000 | 1000 | 1000 | 62.5 |
| Ethylnitrosourea | 1.00 | mM | 1000 | 1000 | 1000 | 1000 |
| Methylmethane sulfonate | 1.00 | mM | 250 | 500 | 500 | 250 |

| (ii) Polycyclic aromatic hydrocarbons | | | | | | |
|---|---|---|---|---|---|---|
| Benzo[a]pyrene | 0.125 | AF | 3.91 | 125 | 125 | 125 |
| 7,12-Dimethylbenz[a]anthracene | 1.00 | mM | 125 | 500 | 250 | 1000 |
| (iii) Aromatic amines | | | | | | |
| Dimethylnitrosamine | 1.00 | mM | 1000 | 1000 | 1000 | 1000 |
| 2-Acetylaminofluorene | 1.00 | mM | 1000 | 1000 | 1000 | 1000 |
| 2,4-diaminotoluene | 1.00 | mM | 1000 | 1000 | 1000 | 1000 |
| IQ (2-Amino-3methylimidazo[4,5-*f*]quinoline) | 1.00 | mM | 1000 | 125 | 1000 | 125 |
| PhIP.HCl (2-Amino-1-methyl-6-phenylimidazo[4,5-*f*]pyridine) | 1.00 | mM | 500 | 1000 | 500 | 1000 |

| (iv) Others | | | | | | |
|---|---|---|---|---|---|---|
| Aflatoxin B1 | 1.00 | mM | 31.25 | 500 | 500 | 500 |
| Cadmium chloride | 1.00 | mM | 31.25 | 125 | 62.5 | 62.5 |
| Cisplatin | 1.00 | mM | 1000 | 1000 | 1000 | 1000 |
| p-chloroaniline | 1.00 | mM | 1000 | 1000 | 1000 | 1000 |

| **II. In vivo genotoxins negative or equivocal in Ames** | | | | | | |
|---|---|---|---|---|---|---|
| Etoposide | 1/0.005 | mM | -/2.5 | 1000/5 | 1000/5 | 1000/5 |
| Hydroquinone | 1.00 | mM | 250 | 1000 | 250 | 1000 |
| Azidothymidine | 1.00 | mM | 1000 | 1000 | 1000 | 1000 |
| Sodium arsenite | 1.00 | mM | 7.81 | 125 | 62.5 | 125 |
| Taxol | 1.00 | mM | 1000 | 500 | 1000 | 1000 |
| Chloramphenicol | 1.00 | mM | 1000 | 1000 | 1000 | 1000 |

**Table 7**

| | | | **SCC** | | **CMFDA** | |
|---|---|---|---|---|---|---|
| | | | **-S9** | **+S9** | **-S9** | **+S9** |
| **(i) Non-carcinogens with negative in vivo genotoxicity data** | highest conc. | | highest conc. <50% | | | |
| | mM | limit | cytotox. over control [µM] | | | |
| Ampicillin trihydrate | 1.00 | mM | 1000 | 1000 | 1000 | 1000 |
| D-mannitol | 1.00 | mM | 1000 | 1000 | 1000 | 1000 |

| **(ii) Non-carcinogens with no in vivo genotoxicity data** | | | | | | |
|---|---|---|---|---|---|---|
| Phenformin HCl | 1.00 | mM | 500 | 1000 | 1000 | 1000 |
| n-butyl chloride | 1.00 | mM | 1000 | 1000 | 1000 | 1000 |
| (2-chloroethyl)trimethyl-ammonium chloride | 1.00 | mM | 1000 | 1000 | 1000 | 1000 |
| Cyclohexanone | 1.00 | mM | 1000 | 1000 | 1000 | 1000 |
| N,N-dicyclohexyl thiourea | 1.00 | mM | 500 | 1000 | 1000 | 500 |
| Trisodium EDTA trihydrate | 1.00 | mM | 500 | 1000 | 1000 | 1000 |
| Ephidrine sulphate | 1.00 | mM | 1000 | 1000 | 1000 | 1000 |
| Erythromycin stearate | 1.00 | mM | 31.25 | 125 | 62.5 | 125 |
| Fluometron | 1.00 | mM | 500 | 1000 | 1000 | 1000 |
| Phenanthrene | 1.00 | mM | 1000 | 1000 | 1000 | 1000 |

| **(iii) Non-genotoxic carcinogens** | | | | | | |
|---|---|---|---|---|---|---|
| D-limonene | 1.00 | mM | 1000 | 1000 | 1000 | 1000 |
| Di-(2-ethylhexyl)phthalate | 1.00 | mM | 1000 | 1000 | 1000 | 1000 |
| Amitrole | 1.00 | mM | 1000 | 1000 | 1000 | 1000 |
| Tert-butyl alcohol | 1.00 | mM | 1000 | 1000 | 1000 | 1000 |
| Diethanolamine | 1.00 | mM | 1000 | 1000 | 1000 | 1000 |
| Melamine | 1.00 | mM | 1000 | 1000 | 1000 | 1000 |
| Methyl carbamate | 1.00 | mM | 1000 | 1000 | 1000 | 1000 |
| Progesterone | 1.00 | mM | 125 | 500 | 62.5 | 250 |
| Pyridine | 1.00 | mM | 1000 | 1000 | 1000 | 1000 |
| Tris(2-ethylhexyl)phosphate | 1.00 | mM | 500 | 1000 | 7.813 | 1000 |
| Hexachloroethane | 1.00 | mM | 1000 | 1000 | 1000 | 1000 |

**Table 8**

| | | | **SCC** | | **CMFDA** | |
|---|---|---|---|---|---|---|
| | | | **-S9** | **+S9** | **-S9** | **+S9** |
| **(i) Non-carcinogens that are negative or equivocal for genotoxicity in vivo** | highest conc. | | highest conc. <50% | | | |
| | mM | limit | cytotox. over control [µM] | | | |
| D,L-menthol | 1.00 | mM | 1000 | 1000 | 1000 | 1000 |
| Phthalic anhydride | 1.00 | mM | 1000 | 1000 | 1000 | 1000 |
| Tertiary-butylhydroquinone | 1.00 | mM | 125 | 1000 | 125 | 1000 |
| o-Anthranilic acid | 1.00 | mM | 1000 | 1000 | 1000 | 1000 |
| 1,3-Dihydroxybenzene (resorcinol) | 1.00 | mM | 1000 | 1000 | 1000 | 500 |
| 2-Ethyl-1,3-hexanediol | 1.00 | mM | 1000 | 1000 | 1000 | 1000 |
| Sulfisoxazole | 1.00 | mM | 1000 | 1000 | 1000 | 1000 |

| **(ii) Non-carcinogens with no in vivo genotoxicity data** | | | | | | |
|---|---|---|---|---|---|---|
| Ethionamide | 1.00 | mM | 1000 | 1000 | 1000 | 1000 |
| Curcumin | 0.125 | AF | 31.25 | 125 | 31.25 | 125 |
| Benzyl alcohol | 1.00 | mM | 1000 | 1000 | 1000 | 1000 |
| Urea | 1.00 | mM | 1000 | 1000 | 1000 | 1000 |

| **(iii) Non-genotoxic carcinogens or carcinogenic by irrelevant (for humans) mechanism** | | | | | | |
|---|---|---|---|---|---|---|
| Sodium saccharin | 1.00 | mM | 1000 | 1000 | 1000 | 1000 |

| **(iv) Supplementary list (prediction of in vitro genotoxicity results less clear)** | | | | | | |
|---|---|---|---|---|---|---|
| Propyl gallate | 1.00 | mM | 500 | 1000 | 1000 | 1000 |
| p-Nitrophenol | 1.00 | mM | 500 | 1000 | 500 | 1000 |
| Sodium xylene sulfonate | 1.00 | mM | 1000 | 1000 | 1000 | 1000 |
| Ethyl acrylate | 1.00 | mM | 1000 | 1000 | 1000 | 1000 |
| Eugenol | 1.00 | mM | 1000 | 125 | 1000 | 125 |
| Isobutyraldehyde | 1.00 | mM | 1000 | 1000 | 1000 | 1000 |
| 2,4-Dichlorophenol | 1.00 | mM | 250 | 500 | 1000 | 1000 |

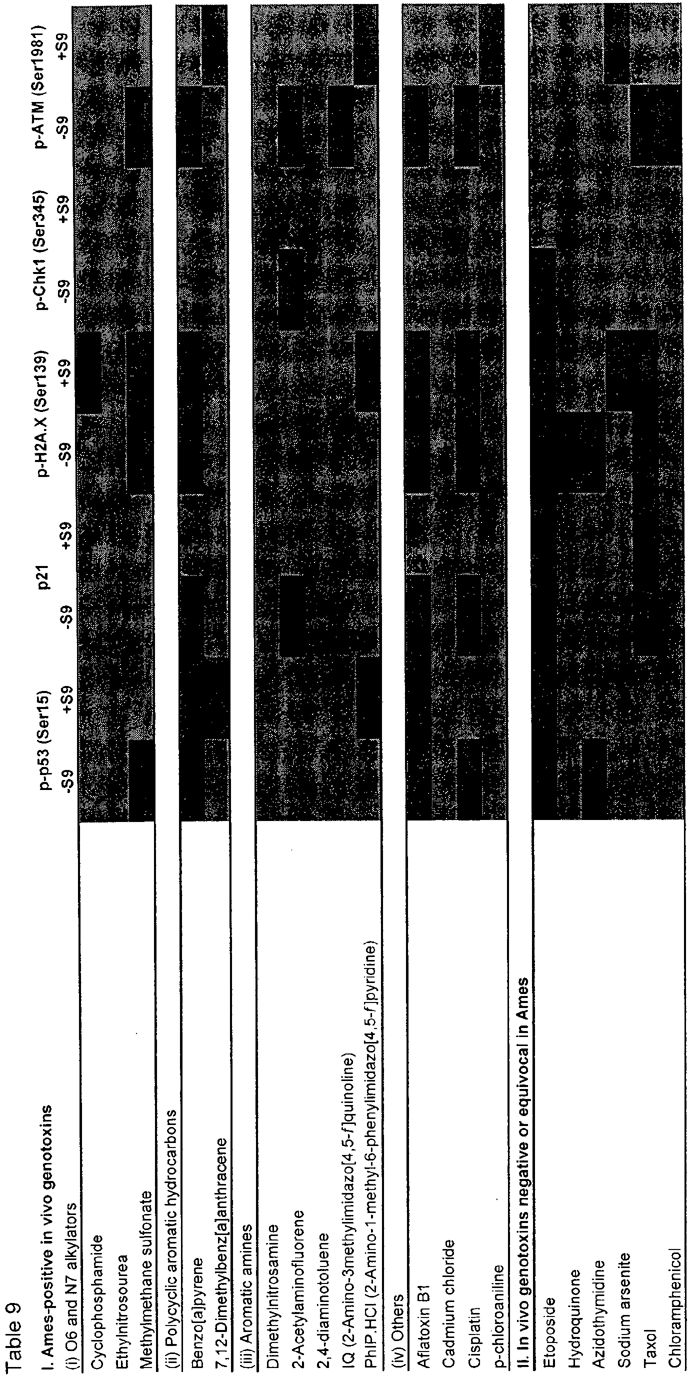

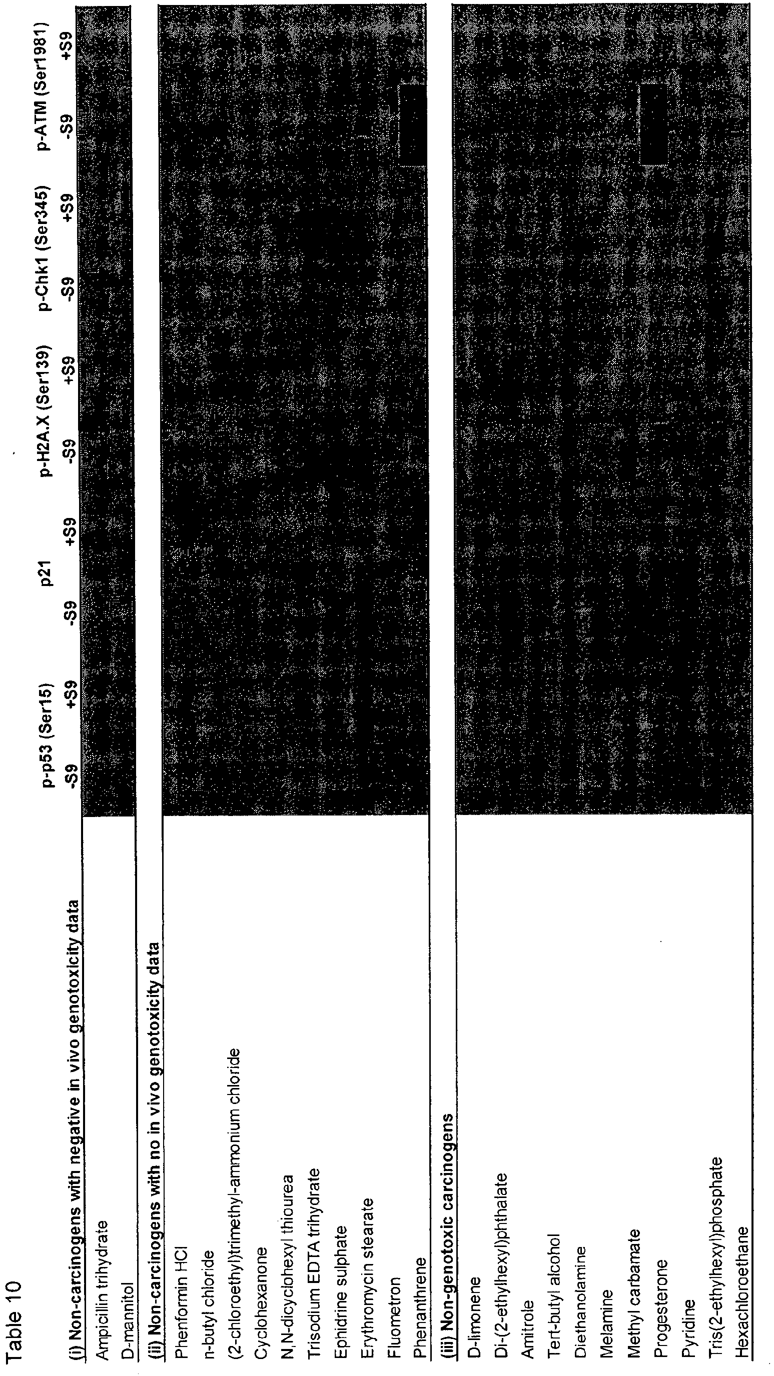

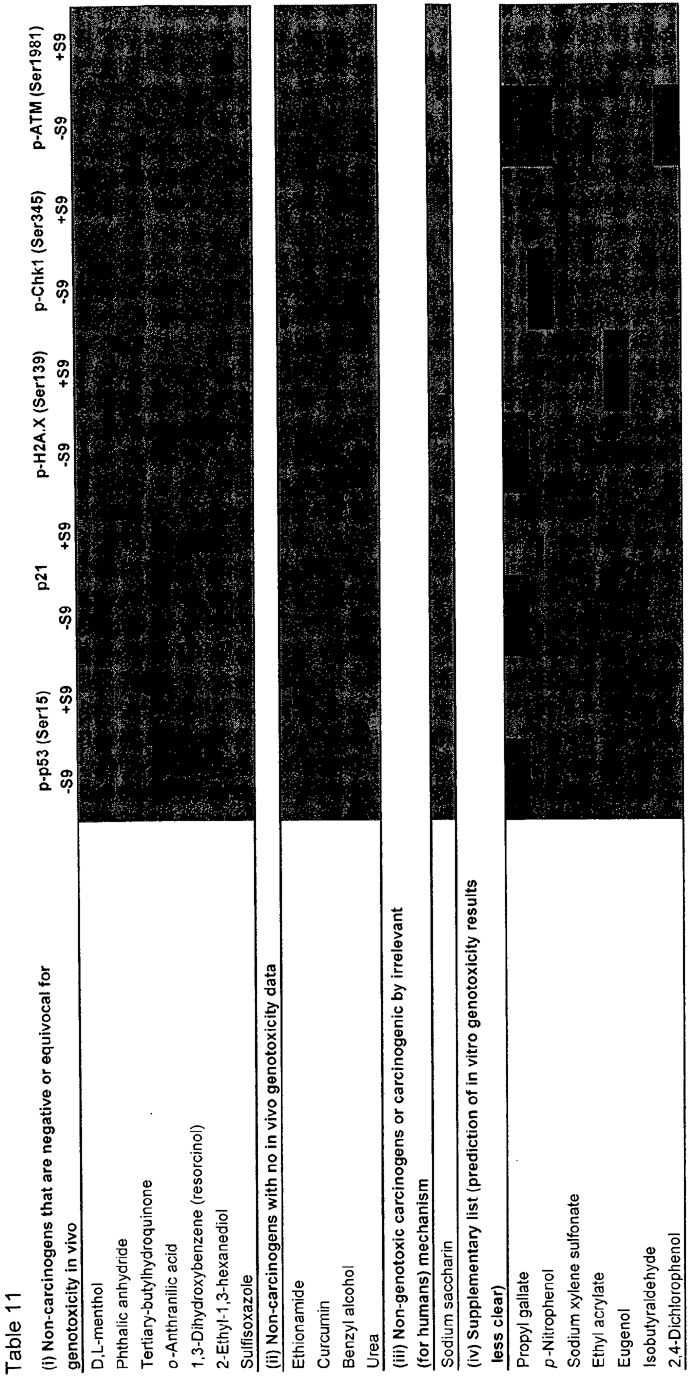

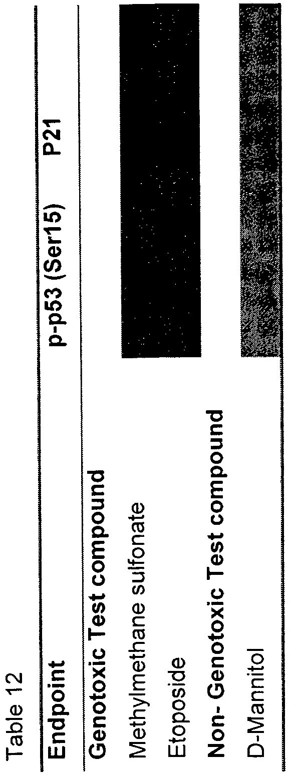

## Claims

1. A method for high-throughput screening of compounds for genotoxic and/or pro-genotoxic activity comprising the step of determining expression levels of active forms of at least the proteins Cellular tumor antigen p53 (p-p53 (Ser15)), Cyclin-dependent kinase inhibitor 1 (p21), Histone H2A.X (p-H2A.X (Ser139)), Serine-protein kinase ataxia telangiectasia mutated (p-ATM (Ser1981)) and Serine/threonine-protein checkpoint kinase 1 (p-Chk1 (Ser345)) in a system incubated with the compounds in comparison with the expression levels in a system not incubated with the compounds, wherein an increase by a factor of at least 1.5 in the expression levels of said proteins in the system incubated with the compounds in comparison with the system not incubated with the compounds indicates said activity with a sensitivity and/or specificity of at least 80%.

2. The method according to claim 1, wherein the expression levels of the proteins p-p53 (Ser15), p21, p-H2A.X (Ser139), p-ATM (Ser1981), p-Chk1 (Ser345), Serine/threonine-protein kinase ataxia telangiectasia mutated and Rad3-related (p-ATR (Ser428)), Cyclin-dependent kinase 1 (p-cdc2 (Thr14/Tyr15)), Growth arrest and DNA damage-inducible protein GADD45 alpha (Gadd45a) and Serine/threonine-protein checkpoint kinase 2 (p-Chk2 (Thr68)) are determined.

3. The method according to claim 1 or 2, wherein the expression levels are determined by immunofluorescence staining or Luminex technology.

4. The method according to any of claims 1 to 3, wherein a decreased concentration of the compounds is administered to provide the expression levels with the factor between 1 and 1.1.

5. The method according to any of claims 1 to 4, wherein the least (pro-)genotoxic compound or a non-(pro-)genotoxic compound is identified, and wherein the least increase or no increase in the expression levels indicates said compound.

6. A method for monitoring the likelihood of developing cancer, tumor, metastasis and/or disorder of angiogenesis, which are caused, mediated and/or propagated by deregulation of damage repair, in response to a treatment with a compound, wherein the expression levels of at least the proteins p-p53 (Ser15), p21, p-H2A.X (Ser139), p-ATM (Ser1981) and p-Chk1 (Ser345) are determined in a biological sample withdrawn from a mammal in need of such treatment with said compound administered to said mammal, wherein an increase by a factor of at least 1.5 in the expression levels of said proteins indicates that said compound has genotoxic and/or pro-genotoxic activity with a sensitivity and/or specificity of at least 80% and said likelihood is increased.

7. An in-vitro method for predicting the likelihood that a patient will suffer from a tumor in response to a therapeutic treatment with a drug, comprising the steps of (i) measuring in a biopsy sample from tissue or plasma of said patient expression levels of at least the proteins p-p53 (Ser15), p21, p-H2A.X (Ser139), p-ATM (Ser1981) and p-Chk1 (Ser345), (ii) exposing ex-vivo a sample from tissue or plasma of said patient to said drug, and (iii) measuring in said exposed sample of step (ii) the expression levels of said proteins specified in step (i) along with calculating the differences in expression levels measured in steps (i) and (iii), wherein an increase by a factor of at least 1.5 in the expression levels of said proteins obtained in this step (iii) compared to step (i) indicates that said drug has genotoxic and/or pro-genotoxic activity with a sensitivity and/or specificity of at least 80% and said likelihood is increased.

8. Use of at least the proteins p-p53 (Ser15), p21, p-H2A.X (Ser139), p-ATM (Ser1981) and p-Chk1 (Ser345) as marker proteins for high-throughput screening of compounds for genotoxic and/or pro-genotoxic activity with a sensitivity and/or specificity of at least 80%.

9. Use of a kit according to the methods and uses of any of claims 1-8 in the detection of genotoxic and/or pro-genotoxic activity comprising nine antibodies, each with specific binding to a different protein selected from the group of p-p53 (Ser15), p21, p-H2A.X (Ser139), p-ATM (Ser1981), p-Chk1 (Ser345), p-ATR (Ser428), p-cdc2 (Thr14/Tyr15), Gadd45a and p-Chk2 (Thr68).

## Patentansprüche

1. Verfahren zum Hochdurchsatz-Screening von Verbindungen auf genotoxische und/oder progenotoxische Aktivität, umfassend den Schritt, bei dem Expressionsniveaus aktiver Formen von mindestens den folgenden Proteinen bestimmt werden, d.h. von zellulärem Tumorantigen p53 (p-p53 (Ser15)), Inhibitor 1 der Cyclin-abhängigen Kinase (p21), Histon H2A.X (p-H2A.X (Ser139)), der bei Ataxie-Telangiektasie mutierten Serinproteinkinase (p-ATM (Ser1981)) und Serin/Threonin-Protein-Checkpoint-Kinase 1 (p-Chk1 (Ser345)), in einem System, das mit den Verbindungen inkubiert wurde, im Vergleich zu den Expressionsniveaus in einem System, das nicht mit den Verbindungen inkubiert wurde, wobei ein Anstieg um einen Faktor von mindestens 1,5 bei den Expressionsniveaus der Proteine in dem System, das mit den Verbindungen inkubiert wurde, im Vergleich zu dem System, das nicht mit den Verbindungen inkubiert wurde, die Aktivität mit einer Empfindlichkeit und/oder Spezifität von mindestens 80% anzeigt.

2. Verfahren nach Anspruch 1, wobei die Expressionsniveaus der Proteine p-p53 (Ser15), p21, p-H2A.X (Ser139), p-ATM (Ser1981), p-Chk1 (Ser345), der bei Ataxie-Telangiektasie mutierten und Rad3-bedingten Serin/Threonin-Proteinkinase (p-ATR (Ser428)), Cyclin-abhängigen Kinase 1 (p-cdc2 (Thr14/Tyr15)), des bei Wachstumsstillstand und DNA-Schaden induzierbaren Proteins GADD45 alpha (Gadd45a) und der Serin/Threonin-Protein-Checkpoint-Kinase 2 (p-Chk2 (Thr68)) bestimmt werden.

3. Verfahren nach Anspruch 1 oder 2, wobei die Expressionsniveaus durch Immunfluoreszenzfärbung oder Luminex-Technologie bestimmt werden.

4. Verfahren nach einem der Ansprüche 1 bis 3, wobei eine verringerte Konzentration der Verbindungen verabreicht wird, so dass die Expressionsniveaus mit dem Faktor zwischen 1 und 1,1 erhalten werden.

5. Verfahren nach einem der Ansprüche 1 bis 4, wobei die am wenigsten (pro-)-genotoxische Verbindung oder eine nicht-(pro-)genotoxische Verbindung identifiziert wird und wobei die geringste Erhöhung oder keine Erhöhung der Expressionsniveaus die Verbindung anzeigt.

6. Verfahren zur Überwachung der Wahrscheinlichkeit, Krebs, Tumor, Metastase und/oder Störung der Angiogenese zu entwickeln, die durch Deregulierung der Schadensreparatur hervorgerufen, vermittelt und/oder verbreitet werden, in Reaktion auf eine Behandlung mit einer Verbindung, wobei die Expressionsniveaus von mindestens den Proteinen p-p53 (Ser15), p21, p-H2A.X (Ser139), p-ATM (Ser1981), und p-Chk1 (Ser345) in einer biologischen Probe bestimmt werden, die einem Säugetier entnommen wird, das eine solche Behandlung mit der dem Säugetier verabreichten Verbindung benötigt, wobei eine Erhöhung der Expressionsniveaus der Proteine um einen Faktor von mindestens 1,5 mit einer Empfindlichkeit und/oder Spezifität von mindestens 80% anzeigt, dass die Verbindung eine genotoxische und/oder progenotoxische Aktivität aufweist, und die Wahrscheinlichkeit erhöht ist.

7. In-vitro-Verfahren zur Vorhersage der Wahrscheinlichkeit, dass ein Patient an einem Tumor leidet, in Reaktion auf eine therapeutische Behandlung mit einem Arzneimittel, umfassend die Schritte, bei denen (i) in einer Biopsieprobe aus Gewebe oder Plasma des Patienten die Expressionsniveaus von mindestens den Proteinen p-p53 (Ser15), p21, p-H2A.X (Ser139), p-ATM (Ser1981), und p-Chk1 (Ser345) gemessen werden, (ii) eine Probe aus dem Gewebe oder Plasma des Patienten dem Arzneimittel ex vivo ausgesetzt wird, und (iii) in der ausgesetzten Probe von Schritt (ii) die Expressionsniveaus der in Schritt (i) angegebenen Proteine gemessen werden sowie die in den Schritten (i) und (iii) gemessenen Unterschiede in den Expressionsniveaus berechnet werden, wobei eine Erhöhung der in diesem Schritt (iii) im Vergleich zu den in Schritt (i) erhaltenen Expressionsniveaus der Proteine um einen Faktor von mindestens 1,5 mit einer Empfindlichkeit und/oder Spezifität von mindestens 80% anzeigt, dass das Arzneimittel eine genotoxische und/oder progenotoxische Aktivität aufweist und die Wahrscheinlichkeit erhöht ist.

8. Verwendung von mindestens den Proteinen p-p53 (Ser15), p21, p-H2A.X (Ser139), p-ATM (Ser1981) und p-Chk1 (Ser345) als Markerproteine zum Hochdurchsatz-Screening von Verbindungen auf genotoxische und/oder progenotoxische Aktivität mit einer Empfindlichkeit und/oder Spezifität von mindestens 80%.

9. Verwendung eines Kits nach den Verfahren und Verwendungen nach einem der Ansprüche 1 bis 8 bei der Erfassung genotoxischer und/oder progenotoxischer Aktivität, umfassend neun Antikörper, jeweils mit spezifischer Bindung an ein anderes Protein, ausgewählt aus der Gruppe p-p53 (Ser15), p21, p-H2A.X (Ser139), p-ATM (Ser1981), p-Chk1 (Ser345), p-ATR (Ser428), p-cdc2 (Thr14/Tyr15), Gadd45a und p-Chk2 (Thr68).

## Revendications

1. Méthode de criblage haut-débit de composés pour une activité génotoxique et/ou pro-génotoxique comprenant l'étape consistant à déterminer les taux d'expression de formes actives d'au moins les protéines d'Antigène tumoral cellulaire p53 (p-p53 (Ser15)), d'Inhibiteur de la kinase dépendante des cyclines 1 (p21), d'Histone H2A.X (p-H2A.X (Ser139)), de Sérine-protéine kinase ATM [« ataxia-telangiectasia mutated »] (p-ATM (Ser1981)) et de Sérine/thréonine-protéine checkpoint kinase 1 (p-Chk1 (Ser345)) dans un système incubé avec les composés par rapport aux taux d'expression dans un système non incubé avec les composés, où une augmentation par un facteur d'au moins 1,5 des taux d'expression desdites protéines dans le système incubé avec les composés par rapport au system non incubé avec les composés est un indicateur de ladite activité avec une sensibilité et/ou une spécificité d'au moins 80%.

2. Méthode selon la revendication 1, dans laquelle les taux d'expression des protéines p-p53 (Ser15), p21, p-H2A.X (Ser139), p-ATM (Ser1981), p-Chk1 (Ser345), de Sérine/ thréonine-protéine kinase ATM et apparenté à Rad3 (p-ATR (Ser428)), de Kinase dépendante des cyclines 1 (p-cdc2 (Thr14/Tyr15)), de Protéine d'arrêt de la croissance et inductible par l'endommagement de l'ADN GADD45 alpha (Gadd45a) et de Sérine/ thréonine-protéine checkpoint kinase 2 (p-Chk2 (Thr68)) sont déterminés.

3. Méthode selon la revendication 1 ou 2, dans laquelle les taux d'expression sont déterminés par marquage par immunofluorescence ou technologie Luminex.

4. Méthode selon l'une quelconque des revendications 1 à 3, dans laquelle une concentration réduite des composés est administrée afin de fournir les taux d'expression avec le facteur compris entre 1 et 1,1.

5. Méthode selon l'une quelconque des revendications 1 à 4, dans laquelle le composé le moins (pro-)génotoxique ou un composé non-(pro-)génotoxique est identifié, et où l'augmentation la plus faible ou une augmentation nulle des taux d'expression est un indicateur dudit composé.

6. Méthode de suivi de la probabilité de développement d'un cancer, d'une tumeur, d'une métastase et/ou d'un trouble de l'angiogenèse, qui sont provoqués, médiés et/ou propagés par la dérégulation des réparations des endommagements, en réponse à un traitement par un composé, où les taux d'expression d'au moins les protéines p-p53 (Ser15), p21, p-H2A.X (Ser139), p-ATM (Ser1981) et p-Chk1 (Ser345) sont déterminés dans un échantillon biologique prélevé chez un mammifère ayant besoin d'un tel traitement par ledit composé administré audit mammifère, où une augmentation par un facteur d'au moins 1,5 des taux d'expression desdites protéines indique que ledit composé possède une activité génotoxique et/ou pro-génotoxique avec une sensibilité et/ou une spécificité d'au moins 80% et ladite probabilité est augmentée.

7. Méthode *in vitro* de prédiction de la probabilité pour un patient de souffrir d'une tumeur en réponse à un traitement thérapeutique par un médicament, comprenant les étapes consistant à (i) mesurer dans un échantillon de biopsie issu d'un tissu ou de plasma dudit patient, les taux d'expression d'au moins les protéines p-p53 (Ser15), p21, p-H2A.X (Ser139), p-ATM (Ser1981) et p-Chk1 (Ser345), (ii) exposer ex *vivo* un échantillon issu d'un tissu ou de plasma dudit patient audit médicament, et (iii) mesurer dans ledit échantillon exposé de l'étape (ii) les taux d'expression desdites protéines spécifiées dans l'étape (i) conjointement avec le calcul des différences de taux d'expression mesurées dans les étapes (i) et (iii), où une augmentation par un facteur d'au moins 1,5 des taux d'expression desdites protéines obtenus dans cette étape (iii) par rapport à l'étape (i) indique que ledit médicament possède une activité génotoxique et/ou pro-génotoxique avec une sensibilité et/ou une spécificité d'au moins 80% et ladite probabilité est augmentée.

8. Utilisation d'au moins les protéines p-p53 (Ser15), p21, p-H2A.X (Ser139), p-ATM (Ser1981) et p-Chk1 (Ser345) comme protéines marqueurs pour le criblage haut-débit de composés pour une activité génotoxique et/ou pro-génotoxique avec une sensibilité et/ou une spécificité d'au moins 80%.

9. Utilisation d'un kit selon les méthodes et les utilisations selon l'une quelconque des revendications 1-8, dans la détection d'une activité génotoxique et/ou pro-génotoxique comprenant neuf anticorps, chacun ayant une fixation spécifique à une protéine différente choisie dans le groupe constitué par p-p53 (Ser15), p21, p-H2A.X (Ser139), p-ATM (Ser1981), p-Chk1 (Ser345), p-ATR (Ser428), p-cdc2 (Thr14/Tyr15), Gadd45a et p-Chk2 (Thr68).
